# EUROPEAN PATENT SPECIFICATION

(11) **EP 4 267 956 B1**
(45) Date of publication and mention of the grant of the patent: **17.06.2026**
(21) Application number: 21845059.1
(22) Date of filing: 27.12.2021
(51) Int. Cl.: G01N 33/487

(54) **A SYSTEM, A METHOD AND A DEVICE FOR SCREENING A DISEASE IN A SUBJECT**
SYSTEM, VERFAHREN UND VORRICHTUNG ZUM SCREENING EINER KRANKHEIT BEI EINER PERSON
SYSTÈME, MÉTHODE ET DISPOSITIF DE DÉPISTAGE D'UNE MALADIE CHEZ UN SUJET

(30) Priority: 28.12.2020 US 202063131188 P
(43) Date of publication of application: 01.11.2023
(73) Proprietor: The Blue Box Biomedical Solutions, S.L., 43144 Vallmoll (Tarragona) (ES)
(72) Inventor: GIRÓ BENET, Judit, 43144 Vallmoll (ES); CHEN, Po-An, Taichung City (TW)
(74) Representative: Torner, Juncosa I Associats, SL
(86) International application number: PCT/EP2021/087672
(87) International publication number: WO 2022/144332

(56) References cited:
- WO-A1-00/79243
- WO-A2-2015/009613
- ES-A1- 2 727 968
- US-A- 6 085 576
- US-A1- 2002 119 513
- US-A1- 2002 152 037
- US-A1- 2004 181 346
- US-A1- 2020 064 291

## Description

### Technical Field

Present invention relates generally to a cancer screening solution. In particular, the present invention relates to a non-invasive and non-irradiating disease screening system, device and method.

### Background of the Invention

As early as Roman times, medicine has paid special attention to human physiological metabolites, e.g., uncontrolled diabetes was historically diagnosed by a sweet taste in urine, liver failure produced a fish-like smell. However, human metabolic studies did not meet the oncology field until April 1989 when Dr. Hywel Williams and Dr. Andres Pembroke from King's College Hospital, London reported a case in the journal The Lancet about a Collie-Doberman owner who attended their practice. She claimed her dog was showing increasing interest in licking a mole in her leg. The mole was then shown to be cancerous and removed, saving the patient's life.

This turning point made it clear that cancer produces metabolic changes in human physiology, thus altering the body's taste, texture, odor. Several publications have been released since then which aim at finding the specific molecule or metabolite in a biological sample that alerts of the presence of cancer in the body - i.e., a cancer biomarker. Examples of such studies include the search for lung cancer biomarkers in a patient's breath, ovarian cancer biomarkers in urine and - indeed- breast cancer biomarkers in urine.

Prior art has one common trait: publications are typically based on the principle that the presence of specific metabolites in a test sample are associated with the diagnosis of a disease, e.g., breast cancer. The described invention, however, does not only focus on the presence of specific components in a sample but to the proportionality between them as well as the comparison between the recent samples and previously analyzed and diagnosed samples.

Likewise, the American Cancer Society predicts that breast cancer represented 30% of all cancers diagnosed in the US in 2020. Breast cancer is considered to be a major cause of mortality in women. However, research dedicated to it is not proportional to its incidence. Actually, the NIH recognized women as underrepresented in medical research. This trend can be observed in the field of oncology, specifically considering current breast cancer screenings. Indeed, a study by the Center for Disease Control and Prevention (CDC) from the US stated that only 65% women attended it in the last 2 years, potentially resulting in 1/3 breast cancers being detected too late, and thus women having a worse prognosis and survival chance. One of many factors that increase the chances of curing the disease is its early detection. It is very important to detect the presence of the disease in early stages which helps in managing and curing the disease. Mammography is currently the gold standard for breast cancer screening, which uses low-energy X-rays to examine the human breast for diagnosis and screening. However, although there is a minimal radiation dose, the continuous exposure to the mammogram can trigger cancer. Moreover, 46% of absenteeism for the mammogram is attributed to pain. Further, the Catalan Healthcare System reported that a 93% of positive results are false positives. All these drawbacks, which are summarized in that the mammography is painful, irradiating and non-sensitive, trigger the need for an improved method for breast cancer diagnosis.

Additionally, there are other breast cancer screening solutions available in the market, such as magnetic resonance imaging (MRI) and echography. However, neither of these two image-based techniques are able to detect all types of breast cancer. Indeed, image-based techniques frequently face difficulty in differentiating breast tumors from healthy dense tissue in women with fibrocystic breasts (>40% women worldwide do actually have fibrocystic breasts). Finally, gene testing can also diagnose one's probability to have a certain type of breast cancer, but this method is extremely expensive.

The present invention is intended to improve the drawbacks of the mammography screening method and other cancer screening solutions available in the market.

Other methods and devices in this field are known by the following patents and patent applications.

WO 2015/009613 discloses a device for analyte detection comprising a sample preparation module, the sample preparation module comprising a volatilization element, in fluid communication with a detection module, the detection module comprising a sensor array comprising a plurality of sensors for detecting a plurality of analytes.

WO2019095790A1 discloses methods and devices for detecting and distinguishing various types of gas molecules or volatile organic compounds (VOCs) indicative of a disease. A sensor array comprising a plurality of sensing electrodes and a reference electrode is used for measuring electric signals indicating the presence or concentration of VOCs from an exhaled breath sample. An algorithm processes the data collected from the array and generates a response pattern which is compared with the response pattern from a healthy subject.

US8366630B2 relates to a system for detecting VOCs derived from a breath sample comprising: an apparatus comprising an array of chemically sensitive sensors of single walled carbon nanotubes coated with non-polar small organic molecules and a processing unit comprising a learning and pattern recognition analyzer where the learning and pattern recognition analyzer receives sensor output signals and compares them to stored data.

US20120326092A1 discloses a method for diagnosing colon cancer in a subject by means of collecting a test breath sample from a test subject; determining the level of at least one VOC from a set of VOCs indicative of colon cancer in the test sample; and comparing the level of the at least one VOC from the test sample with the level of said at least one VOC in a control sample, whereby a significantly different level of said at least one VOC in the test sample as compared to the level of said compound in the control sample is indicative of colon cancer.

US2019271685A1 relates to a system that comprises selected definitive sensor set comprising at least three sensors reactive to the presence of VOCs in an exhaled breath of the test subject, the sensors comprising nanomaterials chosen from metal nanoparticles coated with a first organic coating and single walled carbon nanotubes (SWCNTs) coated with a second organic coating, and a processing unit comprising pattern recognition analyzer, where the pattern recognition analyzer e.g., receives output signals of the sensor set.

US10011481B2 provides an electronic nose device based on specific chemically sensitive field effect transistors. In particular, the sensors of the electronic nose device are composed of non-oxidized, functionalized silicon nanowires which can detect VOCs. Methods of use in diagnosing diseases including various types of cancer are disclosed.

Notwithstanding the above, screening and detection methods and devices known in the art are expensive and complex since they are not designed to be user-friendly. Further, individuals find it difficult to get early cancer screening because of the associated cost and complexity. In 2017, the World Health Organization published the "WHO Position paper on mammography screening" stating that there is an urgent need for a new solution for breast cancer screening. Additionally, breast cancer has been identified by the European Commission as one of the priorities in research. Thus, there exists a need for a non-invasive, inexpensive, sensitive and in-home disease (e.g., cancer) screening solution.

### Description of the Invention

An object of the present invention is to provide a non-invasive and non-irradiating disease screening solution which improves the screening methods and devices known in the art.

The present invention is based on artificial intelligence (AI) applied to the analysis of human samples, which provides a low-cost, user-friendly, non-invasive and perdurable cancer screening solution which can be used in multiple settings, such as within the home settings of its user or in a clinical environment. Further, the present invention is able to run a pain-free and radiation-free quick test in many ways, for instance as a specific self-screening with no need of hospital or trained personnel, or by a healthcare professional, e.g., nurse or gynecologist. Furthermore, because the embedded classification algorithm relies on Al and/or machine learning (ML), the more samples that get analyzed, the higher the classification rate is, and hence the disease can be predicted earlier.

The present invention is able to diagnose cancer without enhancing the risk of developing cancer, unlike mammography. Further, it is radiation-free and non-invasive since cancer can be diagnosed from a routinary urine sample collection, highly sensitive (few cases have been left undiagnosed), precise, accurate, and provides a high reliability (over 95% when classifying metastatic -advanced- breast cancer).

Another advantage over the prior art is that a portable medical screening device suitable for in-home diagnosis is provided, thus reducing the severity of breast cancer in the population due to its early-stage detection. It is an object of the present invention to provide a portable breast cancer diagnosis device capable of diagnosing breast cancer through a simple urine collection, which can be easily carried by a user anytime and anywhere to solve the problems described above. In particular, the portable breast cancer diagnosis device is potentially effective in screening breast cancer at an early stage.

Users can exchange data through a user interface such as a mobile application with the portable medical screening device, which makes it user-friendly and easy to be used without the need of background on medicine or bioengineering. The device can be used indistinctly by different users and indefinitely over time, making it a reusable medical device. Finally, the device is low cost in comparison to the current screening methods that involve very high healthcare expenses and can be used many times and by many users.

The invention is defined by the attached independent claims. Embodiments are described in the dependent claims.

To that end, present invention provides, according to a first aspect, a system for screening a disease, in particular cancer such as breast cancer, in a subject. The system comprises a portable medical screening device and an artificial intelligence-based classification software.

The portable medical screening device has a collection chamber to collect a test sample of urine; a set of chemically sensitive sensors; an analysis chamber to allocate the set of chemically sensitive sensors, the latter being adapted to detect volatile organic compounds (VOCs) in the test sample and to generate an output signal indicative of the presence or absence of VOCs in the test sample as a result of the detection; and a first processing unit operatively connected to the set of chemically sensitive sensors to receive the generated output signal.

The artificial intelligence-based classification software is executed by a remote processing unit (e.g., a cloud-based server) and is configured to determine an outcome regarding a disease by processing and classifying the generated output signal.

The portable medical screening device further includes a communication unit operatively connected to the first processing unit and configured to establish a communication, for instance via Bluetooth, WIFI, a wire-connection, etc., with the remote processing unit.

Thus, the portable medical screening device can connect to the remote processing unit.

The remote processing unit can be accessed via the cited computer device having installed therein the software application.

Present invention also provides, according to a second aspect, a method for screening a disease, particularly cancer, in a subject. The method comprises: collecting, by a collection chamber of a portable medical screening device, at least one test sample of a subject; detecting, by a set of chemically sensitive sensors allocated on an analysis chamber of the portable medical screening device, VOCs in the test sample and generating an output signal indicative of the intensity level (concentration) of VOCs in the test sample as a result of the detection; receiving, by a first processing unit of the portable medical screening device, the generated output signal; and determining, by an artificial intelligence-based classification software executed either by the first processing unit, a software application installed at a computer device or a remote processing unit, an outcome regarding the disease by processing and classifying the generated output signal, wherein if the artificial intelligence-based classification software is executed by the software application or by the remote processing unit, the software application or the remote processing unit are operatively connected to a communication unit of the portable medical screening device.

The terms "subject", "user" and "patient" are used interchangeably herein and refer to any mammalian subject, particularly a human subject, whose samples are screened by the system, device and method of the present invention.

According to the present invention, the first processing unit (or computation component) can process the information on-edge (i.e., on the portable medical screening device itself) or pass the information (i.e., the generated output signal) to the communication unit, which will send the information to the remote processing unit or to the software application, which can also connect to the remote processing unit. The information can therefore be classified on the disclosed portable medical screening device itself or on the software application or on the remote processing unit. The results of the data classification can be displayed via e.g., an output screen on the portable medical screening device, via any user interface at the medical device (such as a color-coded LED-system, an emitted sound) or on the computer device where the software application is installed. In some embodiments, the first processing unit can be a microprocessor and/or a microcontroller such as an Arduino.

### System configurations

In an embodiment, the system according to the first aspect comprises the portable medical screening device and the artificial intelligence (Al)-based classification software, wherein the Al-based classification software is executed by the first processing unit of the device.

In an embodiment, the system comprises the portable medical screening device (which includes the above-mentioned communication unit), the Al-based classification software, and the software application installed on a computer device, wherein the Al-based classification software is executed by the software application. The communication unit is operatively connected to the first processing unit of the device and configured to establish a communication with the software application.

The system comprises the portable medical screening device (which includes the communication unit), the Al-based classification software, and the remote processing unit, wherein the Al-based classification software is executed by the remote processing unit. The communication unit is operatively connected to the first processing unit of the device and configured to establish a communication with the remote processing unit.

In an embodiment, the system comprises the portable medical screening device (which includes the communication unit), the Al-based classification software, the software application installed on a computer device and the remote processing unit, wherein the Al-based classification software is executed by the software application. The communication unit is operatively connected to the first processing unit of the device and configured to establish a communication with the remote processing unit and the software application. Similarly, the software application and the remote processing unit can be operatively connected and communicate between them. In an embodiment, the system comprises the portable medical screening device (which includes the communication unit), the Al-based classification software, the software application installed on a computer device and the remote processing unit, wherein the Al-based classification software is executed by the remote processing unit. The communication unit is operatively connected to the first processing unit of the device and configured to establish a communication with the remote processing unit and the software application. Similarly, the software application and the remote processing unit can be operatively connected and communicate between them. In some embodiments, the portable medical screening device includes a hardware board, which consists of the first processing unit and the communication unit.

### Memory or database, health data and/or demographic data

The system further comprises at least one memory or database configured to store health data and/or demographic data of the subject and/or of a plurality of healthy and/or unhealthy individuals. The memory or database is used by the Al-based classification software and communicates with either the first processing unit, the remote processing unit and/or the software application. Any data contained in the memory or database can be used, extracted and/or updated from a memory or database set without altering the whole memory or database set. This memory or database allows to compare the data therein with the subject's own data in order to translate the comparison result to an outcome.

In an embodiment, the memory or database is included in the remote processing unit. In other embodiments, the memory or database is remote and associated to the remote processing unit and/or to the software application. In other embodiments, the memory or database is included in the software application. In another embodiment, the memory or database is included in the first processing unit.

As explained before, the memory or database can store health data and/or demographic data of the subject and/or of a plurality of healthy and/or unhealthy individuals. The AI-based classification software is further configured to determine the outcome regarding the disease (i.e., the data classification result) considering at least the stored health data and/or demographic data of the plurality of healthy and/or unhealthy individuals and/or of the subject. The memory/database can also store the determined outcome regarding a disease in order to be considered in later screening processes.

The term "health data", in the context of the present invention, refers to any information which relates to the physical or mental health of an individual, and further refers to every type of data related to health status, personal choice about selecting a treatment, health security or policy number, all kind of treatment reports, socio-economic parameters regarding health and wellness, or historical healthcare background such as diseases in past years. Health data refers to, but is not limited to, user's or individual's medical history, family medical history of subjects, medication intake, smoking habit or drug consumption.

The term "demographic data", in the context of the present invention, refers to any information related to the study of a population based on factors such as age, race, sex, etc. Demographic data refers also to socioeconomic information expressed statistically including employment, education, income, marriage rates, birth rates, death rates, etc. Demographic data refers to, but is not limited to, age, gender, sex, ethnic group, place of birth/residence or other habits/ live facts such as frequency of sports practice.

In a particular embodiment, the health data and/or demographic data of the user/plurality of healthy and/or unhealthy individuals can include, but is not limited to, age, sex, gender, ethnicity, place of birth/residence, weight, height, body mass index, habits related to drug consumption (e.g., tobacco, alcohol, cannabis and other toxic substances), user's medical history, family medical history, family cancer history, the user's own cancer history (e.g., breast cancer, lung cancer, prostate cancer, colorectal cancer, melanoma, bladder cancer, kidney cancer, non-Hodgkin lymphoma), histologic type of the tumor, tumor behavior (in-situ or invasive), tumor profile (luminal A, luminal B HER2/neu+, luminal B HER2/neu-, HER2/neu+ or Triple -), tumor size, tumor TNM classification, tumor stage (I, II, III or IV), cancer detection method (via screening or clinically), tumor histological degree (G1, G2 or G3), menstruation, menarche and menopause characteristics (e.g., cycle, timing), number of kids delivered to term, number of premature labors, number of abortions, number of living children, reproductive status (e.g., under fertility medication, pregnant, breastfeeding, post-partum), breast disease history (e.g., breast pain during the menstrual cycle, hormonal changes, swelling, lumpiness or tenderness of the breast, cysts, fibroadenomas, common lumps, nipple discharge, sore/cracked/itchy nipples, inverted nipples), endocrine disorders (e.g., diabetes, thyroid disorders, polycystic ovary syndrome, low testosterone, osteoporosis) or diet type (e.g., omnivore, flexitarianism, "Chicken"-arianism, pescetarianism, vegetarianism, lactovegetarianism, ovovegetarianism, apivegetarianism, veganism, raw veganism, granivorianism, frugivorism, ketogenic diet).

Particularly, the health data and/or demographic data of the user/plurality of healthy and/or unhealthy individuals comprises age, sex, habits related to drug consumption, user's medical history, family medical history, family cancer history, the user's own cancer history, and/or breast disease history.

The memory or database can include different kinds of information according to the screening configuration. For instance, the memory or database can include only health data and/or demographic data of the plurality of healthy and/or unhealthy individuals in the case that the subject's data has not yet been stored in the memory or database, e.g., if it is the subject's first screening. In another example, the memory or database can include the health data and/or demographic data of the plurality of healthy and/or unhealthy individuals as well as health data and/or demographic data of the subject, e.g., when the subject has already introduced its own data from a previous screening and said data was already stored in the memory or database.

The memory or database comprises the health data and/or demographic data of the plurality of healthy and/or unhealthy individuals.

The memory or database comprises the health data and/or demographic data of the subject and of the plurality of healthy and/or unhealthy individuals.

In the present invention, it is understood that the data stored in the memory or database can be raw data and/or processed data. In some embodiments, the data included in the memory or database can be raw data, i.e., data which is not processed by the Al-based classification software. In some embodiments, the data included in the memory or database can be processed data, such as patterns extracted from data after several screenings.

The memory or database comprises stored therein health data and/or demographic data of a plurality of healthy and/or unhealthy individuals, wherein the Al-based classification software comprises determining the outcome regarding a disease considering the stored health data and/or demographic data.

The method also comprises acquiring health data and/or demographic data of the subject and storing the acquired data in a memory or database, wherein the Al-based classification software comprises determining the outcome regarding a disease considering the stored health data and/or demographic data of the subject.

In some embodiments, the health data and/or demographic data is acquired via a user interface of the software application or a portable screening medical device.

### Portable medical screening device

In the present invention, the portable medical screening device can be adapted to enclose different elements to perform the screening. The portable medical screening device enclosing the set of elements is of a size and configuration which makes it to be easily handled and used by an individual, thus enabling an in-home screening.

The portable medical screening device comprises a communication unit operatively connected to the first processing unit and configured to establish a communication with a remote processing unit and/or with a software application installed on a computer device. In an embodiment, the portable medical screening device includes a grid positioned above the collection chamber, and/or a drawer to open, close and support the collection chamber.

In some embodiments, the portable medical screening device can be composed of a box body adapted to enclose the collection chamber, the grid, the analysis chamber, the first processing unit and the communication unit, wherein the box body has a hollow portion for the introduction of the drawer.

Particularly, the portable medical screening device comprises a grid positioned above the collection chamber, and/or a drawer to open, close and support the collection chamber, wherein the portable medical screening device is composed of a box body adapted to enclose the collection chamber, the grid, the analysis chamber, the first processing unit and the communication unit, the box body having a hollow portion for the introduction of the drawer.

The device comprises a collection chamber which collects the human sample which is a urine sample. The test sample can be directly collected into the device or can be collected in a separate apparatus or container and then introduced into the disclosed device.

In some embodiments, the drawer can be filled with an insulating material. In addition, the drawer can comprise a hole (the hole configured to allocate the collection chamber), whose walls are configured to irradiate heat to the sample, where the test sample can be collected. In a particular embodiment, the walls of the whole irradiate heat to the sample by means of a thermal blanket covering the walls.

In the present invention, the portable medical screening device is the element used by a subject to perform the screening. Additionally, according to the above-mentioned system configurations, a software application installed on a computer device, particularly a mobile phone or a tablet, among others, can be further included in the system and configured to establish a communication with the first processing unit of the device. Similarly, a remote processing unit can be further included in the system. The remote processing unit can be located in a cloud or on a computer device such as a PC, a tablet, or a laptop, among others. The communication between the different system elements can be wired or wireless (e.g., via Bluetooth, WIFI, satellite or 5G). The term "computer device" used herein refers to any computer hardware with a central processing unit (CPU) including, but not limited to, a computer, a laptop, a tablet, a personal digital agenda (PDA), a smartphone or a smartwatch.

In an embodiment, the remote processing unit is located in e.g., a cloud, a web-browser, a computer, a laptop, a smartphone or a tablet.

In an embodiment, the software application is located in e.g., a web-browser, a computer, a laptop, a smartphone, a PDA, a tablet or a smartwatch.

In an embodiment, the software application and/or the remote processing unit are wirelessly operatively connected with the first processing unit. In a particular embodiment, the wireless connection is via Bluetooth and/or WIFI.

In a particular embodiment, the medical device can be operated in a remote location. In that case, the medical device has the ability to connect directly to the cloud via satellite or 5G using a special connectivity module embedded in the device specifically for such purpose. In a particular embodiment, the wireless connection is via satellite and/or 5G.

### User interface

In the present invention, the health data and/or demographic data of the subject is collected via a user interface and then processed by the Al-based classification software and/or stored in the memory/database. The user interface can be displayed either by the software application or the portable medical screening device. For instance, the user interface is displayed by the software application installed on a computer device, wherein the computer device is a smartphone.

The software application can be developed using different programming languages including, but not limited to, XML, JavaScript, Swift, Python, SQL, PHP, Ruby, C, C++ or C Sharp. The software application can be run on different operating systems including, but not limited to, Android, iOs, Linux, Mac OS, Windows MS, Ubuntu, Android, Fedora, Solaris, Free BSD or Chrome OS. In a particular embodiment, the software application is run on an Android smartphone and the software application is developed using XML and/or Java. In a particular embodiment, the software application is run on an iOs smartphone, and the software application is developed using Swift.

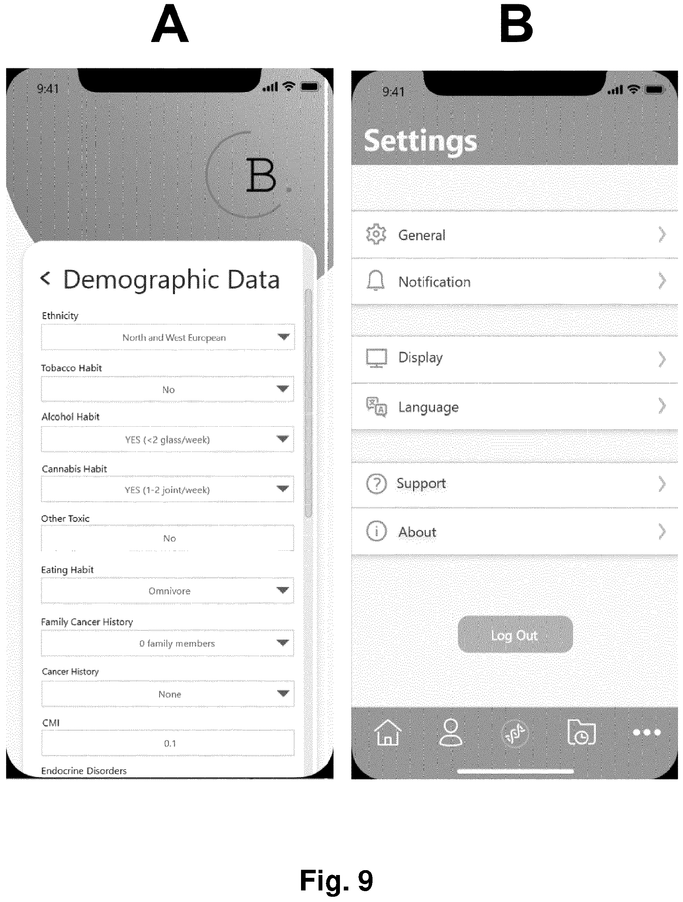

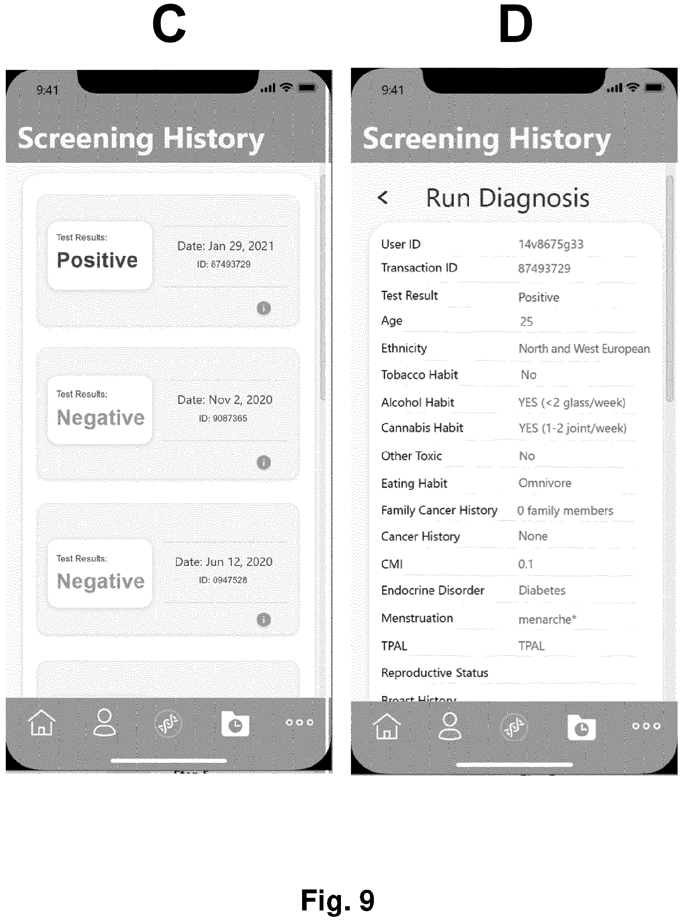

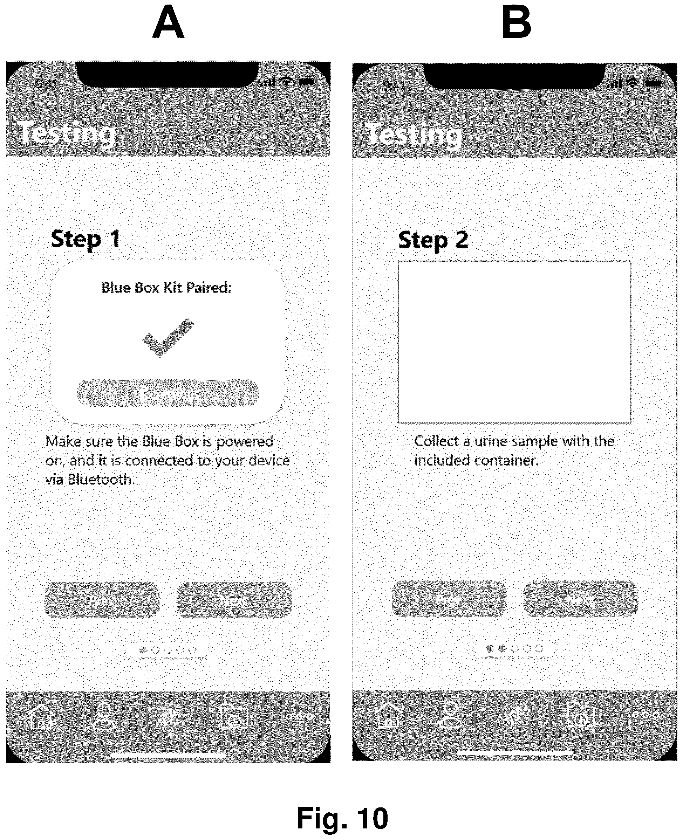

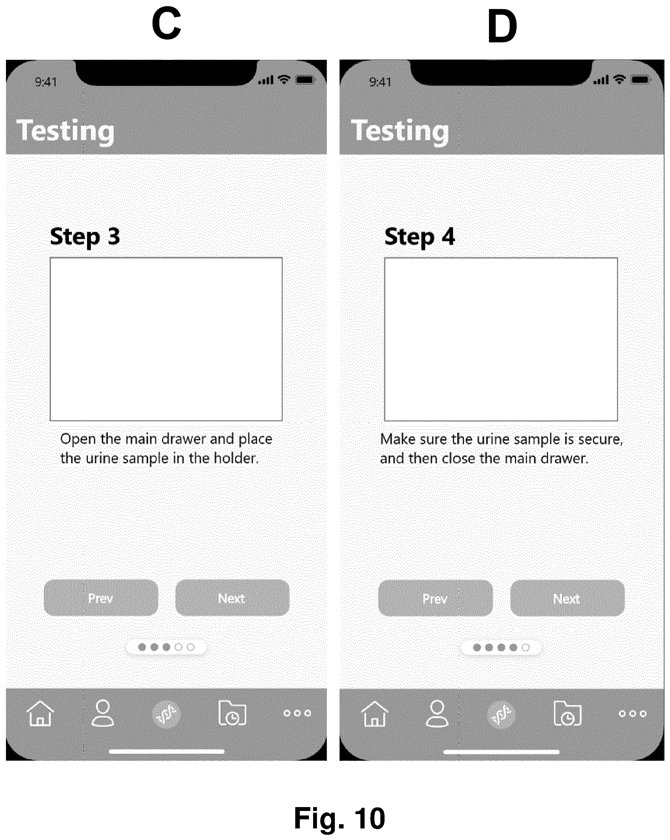

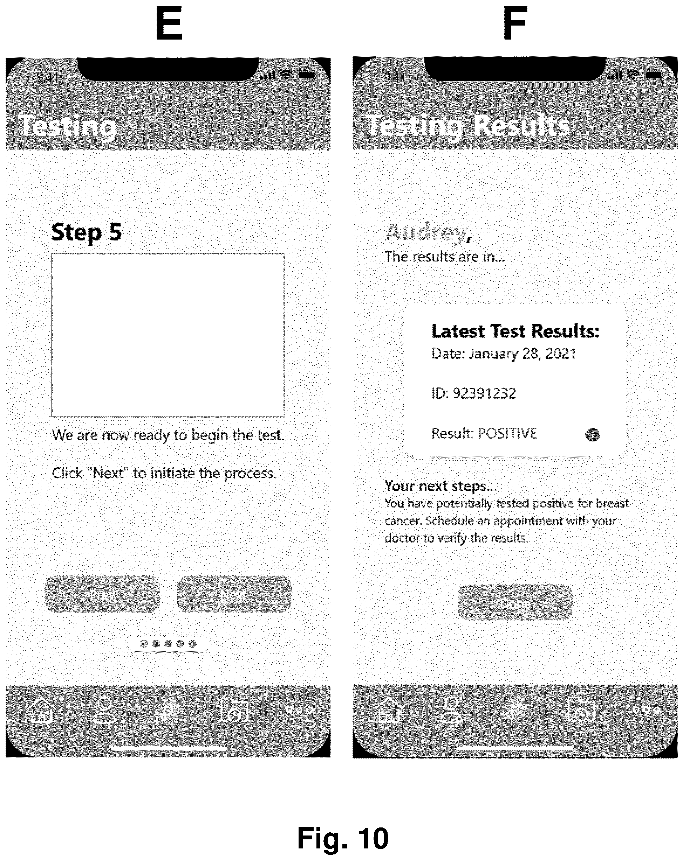

Once the software application is installed on the computer device, the subject can register (i.e., create an account) introducing its personal data, such as the name or nickname, email, birthday, sex and password. After logging in, the software application displays different interfaces, such as a personal information interface (e.g., health data and demographic data of the subject) (Fig. 9 panel A), a screening history interface (Fig. 9 panels C and D), a screening test performance interface (Fig. 10 panels A-F), a configuration/settings interface (Fig. 9 panel B), among others.

By the screening test performance interface, the software application can intuitively guide the subject and/or the healthcare professional through the screening process until the diagnosis is displayed. The subject can decide when to start the screening test performance and the interface displays instructions for the user, for example to enable the Bluetooth, WIFI and/or cellular settings (Fig. 10 panel A) and to introduce the test sample into the portable medical screening device (Fig. 10 panel C). After these instructions, the data will get automatically processed and sent back to the computer device (if data is processed in a processing unit not located in the computer device), which will display the outcome regarding a disease in an understandable way (Fig. 10 panel F). In some embodiments, the software application can guide the subject through the screening process.

The software application can further include several options for the user to receive the outcome regarding the disease, including displaying the outcome in the computer device, receiving a phone call from a healthcare professional (such as a doctor, nurse or psychologist) or setting a face-to-face doctor's appointment, or else the diagnosis can arrive as a notification or a tumor report to the medical personnel.

By the personal information interface, the user can introduce the health data and/or demographic data that will be further considered by the Al-based software to generate an accurate outcome.

In some embodiments, the health data and/or demographic data of the subject is collected via a user interface of the software application and then stored in the memory/database.

Additionally, the software application can incorporate an embedded virtual agent, a chatbot powered by natural language processing (NLP) that continuously performs psychological assessment of the user whilst delivering the outcome. The virtual agent analyses the user's text-based responses (e.g., via a chatbot inbuilt in the software application) and determines if the user is processing their emotions accordingly or following avoidant behavior patterns, hence needing psychological assistance. Alternatively or additionally, the virtual agent can analyze the user's facial expression (captured by the computer's camera) and process it using convolutional neural networks to correlate them with the emotion that the user might be feeling.

Different software applications can be used depending on the environment in which the screening is performed. In an embodiment, when the screening is intended to be performed in-home, the software application is directed to the subject and includes e.g. a forum for multiple purposes (e.g., social networking, scientific articles sharing or healthcare data sharing). In another embodiment, the screening is intended to be performed in-home by a subject with IT limited capacities (e.g., an elder) being the software application simplified and mainly including the outcome display. In yet another embodiment, the screening is intended to be performed in-home, the software application being directed to the subject and not including the outcome display, but rather the screening history of the subject (the outcome is sent to a healthcare professional). In another embodiment, the screening is intended to be performed in a clinical environment (e.g., hospital, nursing home, pharmacy, ambulance), the software application being installed in a PDA or tablet, managed by a healthcare professional and mainly including the outcome display.

### Chemically sensitive sensors

In the present invention, the portable medical screening device includes a set of chemically sensitive sensors that detects the presence or absence of VOCs in the test sample, in which the first processing unit is operatively connected to, in order to receive the generated output signal. In a particular embodiment, the first processing unit is connected to the set of chemically sensitive sensors via a wired connection.

Present invention is based on the principle that the presence of breast cancer, is not denoted by a certain chemical compound found in the urine, but by the proportionality between all the chemical compounds found in the urine. The set of chemically sensitive sensors at the analyzing chamber is not specific for some biomarkers, e.g., breast cancer biomarkers, but rather sensitive to a plurality of sample characteristics or compositions. Additionally, sample classification does not simply rely on the presence or absence of a specific set of e.g., cancer biomarkers.

The chemical compounds that are captured by the chemically sensitive sensors are not direct e.g., cancerous metabolites. Instead, they are the residual metabolites that result from cell metabolism. Because e.g., cancer causes a change in the physiology of the body, the aforementioned metabolites vary in concentration due to cancer. This strategy of targeting a proportion between chemicals instead of a specific biomarker is inspired after the dog's olfactory cortex.

According to the invention, the generated output signal is indicative of the presence or absence of VOCs in the test sample. In an embodiment, the generated output signal comprises an electric signal whose voltage is proportional to a concentration of the VOCs. For instance, the output signal is '0' if VOCs are not present, '1023' if the test sample is saturated with the VOCs, and the signal will take an intermediate value if there is some quantity of VOCs. That is, the output signal is, particularly an analog signal.

In an embodiment, the set of chemically sensitive sensors comprises photoionization detectors (PID), flame ionization detectors (FID), metal oxide sensors (MOS) and/or Silicon Photonic Ring Resonator (SPRR), or any type of gas sensing structure that is sensitive to Volatile Organic Compounds and consists of a Self-assembled Monolayer Coated Sensor Array with Concentration-independent Fingerprints. In an embodiment, the set of chemically sensitive sensors comprises metal oxide sensors and/or organic compounds sensitive sensors (such as semiconductor-based chemirresistive sensors, electrochemical sensors, metal oxide sensors and PID sensors). In accordance with the invention, the MOS is a tin oxide sensor.

These sensors can be used in combination -or not- with nanotechnology or microfluidics techniques, such as having the human fluid circulating through a microfluidic circuit before or during the analysis and measuring its ability to flow through it additionally to measuring its smell. In an embodiment, the set of chemically sensitive sensors is used in combination with microfluidics techniques. In an embodiment, the set of chemically sensitive sensors is used in combination with nanotechnology techniques.

The portable medical screening device is composed of a set of chemically sensitive sensors.

Particularly, the portable medical screening device is composed of one to twenty chemically sensitive sensors. More particularly, the portable medical screening device is composed of five to ten chemically sensitive sensors. More particularly, the portable medical screening device is composed of nine chemically sensitive sensors.

The set of chemically sensitive sensors can be configured to detect the VOCs all at the same time. In other embodiments, each one of the chemically sensitive sensors can be configured to detect the VOCs at a given time interval. Likewise, in some embodiments, each one of the chemically sensitive sensors can react to one or more VOCs.

In an embodiment, the set of chemically sensitive sensors detect the VOCs for various minutes, preferably below 45 minutes. In a particular embodiment, the set of chemically sensitive sensors detect the VOCs during a time interval between 10 and 120 minutes, preferably a time interval between 25 and 35 minutes.

### Test sample

In one embodiment, apart from detecting the presence or absence of VOCs, the portable medical screening device further detects some characteristics of the test sample, such as the chemical composition, the smell, the temperature, the viscosity (i.e., the Reynolds number), the density, the color, the conductivity, the capacitance, the boiling time, the weight or translucency thereof, the refractive index, the change of any chemical/physical property along time or while the sample is being heated up, among other physical/chemical properties.

In some embodiments, it might be necessary to apply some chemical or physical stimulus to the sample, such as but not limited to, a chemical reagent or any other substance, a physical vibration, such as sonification, heat, pressure, movement, such as decantation of the substrate, and other stimuli. Additionally, the observation can also be made based on the change in the aforementioned observation or the velocity at which the change happens.

In another embodiment, the portable medical screening device generates a specific response (i.e., pattern) given the nature of the test sample or given a specific smell of the test sample. The present invention can analyze at least one test sample to generate the output signal indicative of the presence or absence of VOCs.

The test sample is urine.

In some examples, the drawer is filled with an insulating material and comprises a hole (the hole configured to allocate the collection chamber), thus the collection chamber containing the sample can be introduced into the hole and be better retained. Moreover, the walls of the hole can further irradiate heat to the sample. In some embodiments, this is done by the inner side of the hole walls being covered with a thermal blanket (i.e., a dissipative resistance that converts electric energy into heat). The thermal blanket thus can heat up the walls of the hole, which are directly in contact with the sample or else with the collection chamber, and therefore transfer the heat to the collection chamber and the sample. To control the sample heating process, in an embodiment, the first processing unit is operatively connected to a relay that can mechanically start and stop the system from feeding an electric voltage to the thermal blanket and to a temperature sensor in contact with the hole walls. The temperature sensor can be integrated within the thermal blanket, directly placed in contact with the hole walls, in contact with the collection chamber, or directly in contact with the sample. The temperature sensor is operatively connected with the first processing unit, which reports the temperature (or an approximation) at which the sample is at every instant of time, in a continuous fashion. Based on this information, the first processing unit is configured to make a decision to keep the thermal blanket on or off and thus control the temperature of the samples as well as the velocity at which it heats up. By doing so, the thermal blanket, the temperature sensor, the relay and the first processing unit form a feedback control loop to set the temperature of the sample.

In an embodiment, the set of chemically sensitive sensors are configured to detect the VOCs in the test sample once a temperature of the test sample hits a temperature of at least 25 ºC. Particularly, the test sample hits a temperature of at least 40 ºC. Particularly, the test sample hits a temperature of at least 50 ºC. In yet another particular embodiment, the test sample hits a temperature between 25 ºC and 90 ºC. In yet another particular embodiment, the test sample hits a temperature between 40 ºC and 90 ºC. Accuracy and optimization of the screening is improved when the test sample hits such temperatures. The chemically sensitive sensors stop detecting the VOCs once the temperature in the test sample hits a temperature of e.g., 80-95 ºC depending on the selected temperature to heat the sample.

The test sample can be directly collected into the portable medical screening device or can be collected in a separate apparatus or container and then introduced into the disclosed device. In a particular embodiment, the container is the collection chamber.

### Artificial intelligence-based classification software

The artificial intelligence-based classification software can comprise one or more machine learning/artificial intelligence-based (ML/AI), or statistics classification algorithms, including but not limited to, artificial recurrent neural network (RNN) -such as long short-term memory (LSTM)-, artificial neural network (ANN), convolutional neural network (cNN or ConvNet), principal component analysis (PCA), multi-layer perception (MLP), generalized regression neural network (GRNN), fuzzy inference systems (FIS), self-organizing map (SOM), radial bias function (RBF), genetic algorithms (GAS), neuro-fuzzy systems (NFS), adaptive resonance theory (ART), partial least squares (PLS), multiple linear regression (MLR), principal component regression (PCR), discriminant function analysis (DFA), linear discriminant analysis (LDA), cluster analysis, and nearest neighbor. In particular, the Al/ML classification algorithm is an RNN such as LSTM and/or an ANN.

Additional algorithms suitable for identifying patterns of VOCs and quantifying their concentration can include, but are not limited to, Fisher linear discriminant analysis (FLDA), soft independent modeling of class analogy (SIMCA), k-nearest neighbors (kNN), and fuzzy logic algorithms. In some embodiments, the FLDA and canonical discriminant analysis (CDA) and combinations thereof are used to compare the output signature and the available data from the database.

Many of the algorithms are neural network-based algorithms. A neural network has an input layer, processing layers and an output layer. The data extracted by the sensors from the sample is fed as an input to the input layer and flows through the processing layers until it reaches the output layer. At every processing layer, the information is modified and passed on to the next layer. The information that reaches the output layer leads to the outcome of the classification. The processing layers are made up of nodes that simulate neurons by the interconnection to their nodes. In operation, when a neural network is combined with a sensor array, the sensor data is propagated through the networks. In this manner, a series of vector matrix multiplications are performed, and unknown analytes can be readily identified and determined. The neural network is trained by correcting the false or undesired outputs from a given input. Similar to statistical analysis revealing underlying patterns in a collection of data, neural networks locate consistent patterns in a collection of data, based on predetermined criteria.

### Outcome regarding a disease

In the present invention, once the Al-based classification software has processed all the inputs (e.g., data obtained by the sensors and user's data introduced by the software application), the software application or the portable medical screening device can display the outcome to the user.

The term "outcome" herein refers to the determination or detection of a disease (e.g., diagnosis of a disease), which can comprise an indication regarding the presence or absence of the disease, a risk probability of suffering from the disease, an indication of a degree and/or of a type (or subtype) of the disease and/or a size of a tumor, a recommendation for a certain treatment, a prediction of the evolution of a certain disease, among others.

In some embodiments, if the sample classification is performed by the Al-based classification software which is hosted at the software application, the outcome is shown at the computer device. In another embodiment, if the sample classification is performed by the Al-based classification software which is hosted at the software application, the outcome is sent via the internet, Bluetooth, or another means and shown at the portable screening device. In another embodiment, if the sample classification is performed by the Al-based classification software which is hosted at the screening device or at the cloud-based server, the outcome can be sent, for instance via internet or Bluetooth, from such screening device or from the cloud server to the software application, where they will be displayed. In another embodiment, if the sample classification is performed by the Al-based classification software which is hosted at the cloud-based server, the outcome can be sent, for instance via internet or Bluetooth, from such screening device to the portable screening device, where they will be displayed. In yet another embodiment, if the sample classification is performed by the Al-based classification software which is hosted at the first processing unit, the outcome is shown at the screening device. This allows a user to check the outcome as soon as the classification is completed.

In an embodiment, the Al-based classification software is configured to determine an outcome regarding the disease by processing and classifying the generated output signal. In some embodiments, the outcome regarding the disease comprises an indication regarding the presence or absence of the disease. For instance, the outcome might be of the form of a report or a binary outcome, e.g., values "0" or "1", relative to "cancer" and "healthy", etc. The Al-based classification software can determine the outcome through a risk probability of suffering from the disease. In some embodiments, the outcome regarding the disease is a risk probability of suffering from the disease.

Moreover, artificial intelligence is also implemented so as to provide information about the test sample under analysis: size of the tumor (if applicable), type of cancer (if applicable) and other information. In a particular embodiment, the outcome comprises an indication of a degree and/or of a type (or subtype) of the disease and/or when the disease is cancer, of a size of a tumor. The term "disease" can also include any condition, disorder, syndrome, or state of the subject, which is altered in comparison to a healthy subject, and refers to a particular abnormal condition that negatively affects the structure or function of all or part of an organism. Conditions or altered states of the subject are often associated with specific signs and symptoms which can trigger a disease, disorder, syndrome, or a severe condition.

In accordance with the invention, the disease is breast cancer.

The present invention also relates to the identification of patterns involved in cancer progression. In particular, the invention is capable of identifying different grades of cancer within and between stages thereof. Broadly defined, these stages are non-malignant versus malignant, but may also be viewed as normal versus atypical (optionally including reactive and pre-neoplastic) versus cancerous. Another definition of the stages is normal versus precancerous (e.g., atypical ductal hyperplasia (ADH) or atypical lobular hyperplasia (ALH)) versus cancerous (e.g., carcinoma in situ such as DCIS and/or LCIS) versus invasive (e.g., carcinomas such as IDC and/or ILC). The invention may also be applied to discriminations between normal and non-normal (including cancerous and other non-normal cells).

Grading of e.g., breast cancer is normally done for cases of invasive ductal carcinoma (IDC), and may be done for invasive lobular carcinoma (ILC) as well, where cytological criteria such as the Nottingham BSR, nuclear morphology, tissue architecture, proliferation index (such as assays for PCNA or Ki67), and extent of differentiation are used to assign a grade of I, II or III to particular breast cancer samples. Grade I is usually where the cells are still well differentiated and are usually positive for the estrogen receptor (ER). Grade III is usually where the cells are poorly differentiated and usually negative for ER. Grade II is generally where the cells have characteristics intermediate between grades I and III and can make up approximately 60% of all samples assayed.

A "stage" or "stages" (or equivalents thereof) of e.g., breast cancer refer to a physiologic state of a e.g., breast cell as defined by known cytological or histological procedures (including immunohistology, histochemistry and immunohistochemistry). Non-limiting examples include normal versus abnormal, non-cancerous versus cancerous, the different stages described herein (e.g., hyperplastic, carcinoma, and invasive), and grades within different stages (e.g., grades I, II, or III or the equivalents thereof within cancerous stages).

In an embodiment, the outcome regarding the disease comprises an indication of a degree of the disease, such as the stage, histological grade, histological type, behavior, profile, TNM staging (tumor/nodes/metastases) of the disease. In a particular embodiment, the stage regarding the disease can be I, II, III or IV. In a particular embodiment, the behavior regarding the disease can be hyperplastic, carcinoma or invasive. In a particular embodiment, the histological grade regarding the disease can be grade I, grade II or grade III. In a particular embodiment, the profile regarding the disease can be Luminal A, Luminal B HER2/neu+, Luminal B HER2/neu-, HER2/neu+ or Triple -.

The subject can use the present invention to diagnose several diseases in one screening. Accordingly, in an embodiment, the outcome regarding the disease comprises at least one disease. In a particular embodiment, the outcome comprises an indication of a type of the disease. In a particular embodiment, breast cancer, pancreatic cancer, lung cancer, colon cancer, ovarian cancer, liver cancer and uterus cancer can be diagnosed. In a particular embodiment, breast cancer, pancreatic cancer and lung cancer can be diagnosed.

Tumor volume is important for treatment planning and assessment of the degree of tumor regression and disease prognosis. Therefore, the present invention is also capable of determining the size and volume of the tumor itself. In an embodiment, the outcome regarding the disease comprises an indication of the size of a tumor. In a particular embodiment, the disease is cancer, and the outcome comprises an indication of the size of a tumor.

Additionally, it can comprise treatment recommendations, based on an observation of treatments performed in the past and their success rates. In an embodiment, the outcome regarding the disease comprises a treatment recommendation.

In some embodiments, the outcome regarding the disease is a prevision of the evolution of the disease based on the comparison between the current sample and previously analyzed samples.

The present invention can be used for screening a disease and for monitoring purposes. For instance, a patient diagnosed with breast cancer, can be screened periodically to obtain a diagnosis regarding the stage/grade of the disease at different timepoints. In some embodiments, the present invention can be used to monitor disease progression, regression, recurrence, and/or response to treatment in a subject.

In a particular embodiment, the present invention is further used for monitoring the recurrence of a tumor in a subject. In one embodiment, the invention provides a clinical algorithm for personalized monitoring of the course of a tumor and its treatment in a given patient. The monitoring can be prior to treatment, during treatment, or following treatment (e.g., following surgery, radiation and/or chemotherapy). Thus, the system and method of the invention can be used to monitor the progression, spread, treatment, metastasis, and/or recurrence of the tumor.

In an embodiment, the outcome regarding the disease has an accuracy (i.e., classification rate) of at least 75%. In a particular embodiment, the outcome regarding the disease has an accuracy of 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100%. In a particular embodiment, the outcome regarding the disease has an accuracy of at least 95%. In a particular embodiment, the outcome regarding the disease has an accuracy of 95%, 96%, 97%, 98%, 99% or 100%. In another embodiment, the % of accuracy is 100%.

In an embodiment, the outcome regarding the disease has a sensitivity of at least 75%. In a particular embodiment, the outcome regarding the disease has a sensitivity of 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100%. In a particular embodiment, the outcome regarding the disease has a sensitivity of at least 95%. In a particular embodiment, the outcome regarding the disease has a sensitivity of 95%, 96%, 97%, 98%, 99% or 100%. In another embodiment, the % of sensitivity is 100%.

In an embodiment, the outcome regarding the disease has a specificity of at least 75%. In a particular embodiment, the outcome regarding the disease has a specificity of 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100%. In a particular embodiment, the outcome regarding the disease has a specificity of at least 95%. In a particular embodiment, the outcome regarding the disease has a specificity of 95%, 96%, 97%, 98%, 99% or 100%. In another embodiment, the % of specificity is 100%.

### Embodiments of the screening method

The step of detecting VOCs in the test sample refers to the determination of the presence or absence of VOCs depending on the concentration of VOCs, i.e., intensity to which some VOCs are present in the test sample and the proportionality between their intensities. Each sensor of the set of chemically sensitive sensors outputs an electrical signal whose intensity is proportional to the concentration of various VOCs in the sample. Each sensor has a different affinity to a range of VOCs. Some VOCs can be detected by more than one sensor. Hence, the combined response of all sensors is informative about the composition of the sample. The concentration of VOCs is analyzed at each timepoint of the sensing period, which can last e.g., 30 minutes while the sample is heated up. The sample headspace is all the space inside the sample container (or inside the drawer, if no sample container is used), i.e., analysis chamber, that is not occupied by the sample. Before the analysis starts, the sample headspace is filled with ambient air at room temperature. During the analysis, while the thermal blanket transfers heat to the sample container and/or the sample, the VOCs contained in the sample evaporate at their specific evaporation temperature. The sample and the sample headspace therefore change in composition over time, as the VOCs evaporate from the sample and become present in the sample headspace. The sensor's response hence varies over time. Typically, VOCs consisting of smaller volatile molecules evaporate before. An electric output signal is generated from the information extracted during the sensing period, which is indicative of the concentration of VOCs in the test sample as a result of the detection. For instance, the output signal is '0' if VOCs are not present, '1023' if the test sample is saturated with VOCs, and the signal takes an intermediate value if there is some quantity of VOCs. That is, the electric output signal is, particularly, an analog signal.

The generated electric output signal is then sent to the first processing unit via a wire or wireless connection. Once the first processing unit has received the VOCs data extracted from the test sample, this information needs to be processed to determine an outcome regarding the disease by processing and classifying the generated output signal through an Al-based classification software.

Before making use of the Al-based classification software, the system checks for the latest version of the classification software at a version bucket. Before any classification, the system makes sure that the latest version of the classification algorithm is installed. The Al-based classification software periodically receives real-time updates.

The Al-based classification software comprises different algorithms that use different data to generate the outcome. For instance, an algorithm is responsible for processing the health data and demographic data of the subject taken from the software application installed on a computer device; an algorithm is responsible for processing the data obtained from the set of chemically sensitive sensors; and an algorithm which merges all the input data and recognizes patterns among the information stored in the memory or database.

In an embodiment, the Al-based classification software comprises at least one ML/Al-based classification algorithm. In a particular embodiment, the Al-based classification software comprises an ANN that takes as input the health data and demographic data of the subject. In a particular embodiment, the Al-based classification software comprises a LSTM network that takes as input the sensor data.

The input consists e.g., of a 2D data array. Every row of the array is a 1D vector that contains the response of one sensor. Every element in each column is related to an instant of time. Hence, the data array has 9 rows (one per sensor) and as many columns as times that the sample is odorized which can be e.g., 100 columns.

In the present invention, the determined outcome is stored in a memory or database. In this case, the Al-based classification software, in subsequent screening operations, can also take into account the first, or initial, screening result of the subject to determine further screening results thereof. Thus, in an embodiment, the method further comprises storing the determined outcome regarding the disease in a memory or database, wherein the artificial intelligence-based classification software comprises determining the outcome regarding the disease considering the stored outcome.

For every new analysis, the classification algorithm reaches an output that is determined by whether the input signal resembles more to the previously analyzed diseased samples or to the control (healthy) samples. This is possible because the Al algorithm in the classification software has been previously trained: the weights of every neuron in the network have been adjusted to minimize discriminant error, i.e., the difference between a predicted probability of having cancer and the absolute truth (0% or 100% for every sample, as determined by a biopsy carried out after a portable medical screening device analysis).

Additionally, instead of generating knowledge about a test sample based uniquely on their composition, the present invention in some embodiments proposes that every time a new test sample is analyzed, and some data is obtained from the human sample by the screening device, the obtained data is compared to all data in the database, i.e., sensor data from all previously-analyzed samples, patient data that the user has previously input via the user interface and/or sensor/patient data from other users. The classification of the new test sample is particularly assessed based on the degree to which it resembles previously analyzed data. Its class depends on the degree to which the new test sample resembles previously analyzed cancerous samples and the degree to which the new test sample resembles previously analyzed control (healthy) samples. In other words, machine learning is implemented so as to classify the test samples.

Thus, the present invention provides a portable screening solution that can be used to diagnose cancer using different types of test samples from a subject. The disclosed device can be used multiple times by one single subject or by multiple subjects.

The previous and other advantages and features will be more fully understood from the following examples and detailed description of embodiments, with reference to the attached figures, which must be considered in an illustrative and non-limiting manner.

### Brief Description of the Drawings

Fig. 1 shows an example of the portable medical screening device, according to an embodiment.
Fig. 2A shows an example of the inner structure of the portable medical screening device, according to an embodiment. Figs. 2B and 2C show an example of the positioning of the chemically sensitive sensors inside the inner structure of the portable medical screening device, according to an embodiment.
Fig. 3 shows a system for screening a disease in a subject, according to an embodiment.
Fig. 4 shows different examples where the Al-based classification software can be executed.
Fig. 5 shows another example of the proposed system.

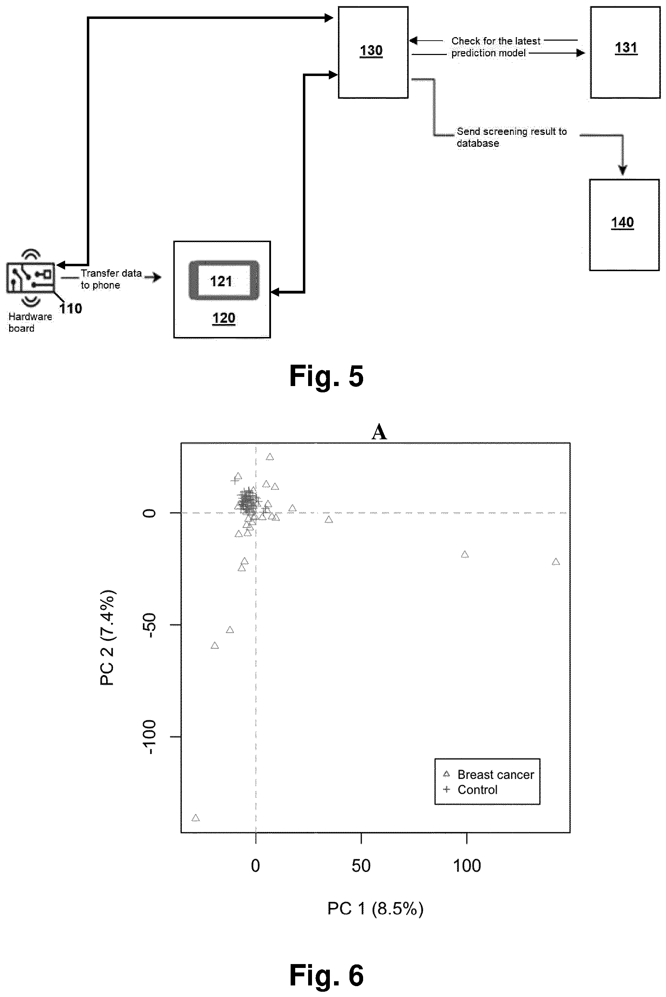

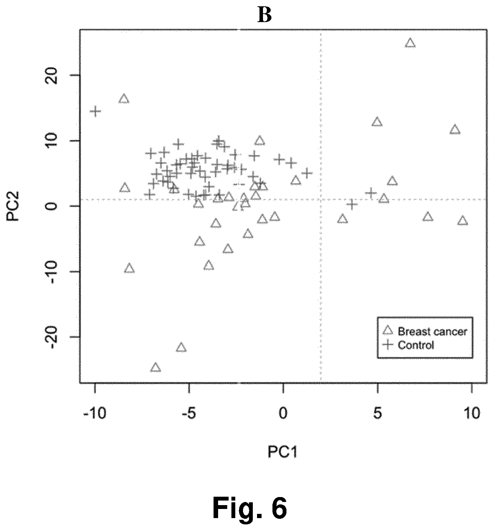

Fig. 6 panel A shows a score plot including control (crosses) and breast cancer (triangles) human urine samples projected against an imaginary plane which maximizes sample variability between classes. The imaginary plane is defined by two vectors: principal component (PC) 1 and PC 2. These two vectors represent a linear combination of different features. These samples have been analyzed using a gas chromatograph-mass spectrometer. Fig. 6 panel B shows a zoom into the area where most samples are laying. Quadrant I (top left) contains 60 samples 49 of which are controls (81.7%). Quadrants II and III (top right and bottom right, respectively) contain 12 breast cancer samples out of a total of 14 (85.7%). Quadrant IV (bottom left) presents 16 breast cancer samples.
Fig. 7 panel A shows a distribution of control and breast cancer human urine samples after device odouring. These samples have been analyzed with the portable medical screening device of the present invention. The imaginary plane is defined by two vectors: PC 1 and PC 3. Samples have been projected against their highest variability plane. Fig. 7 panel B shows a zoom into the region of interest. A tendency for breast cancer samples to cluster below an imaginary grey dotted line is observed. Control samples show a more disperse and higher-variability fashion.

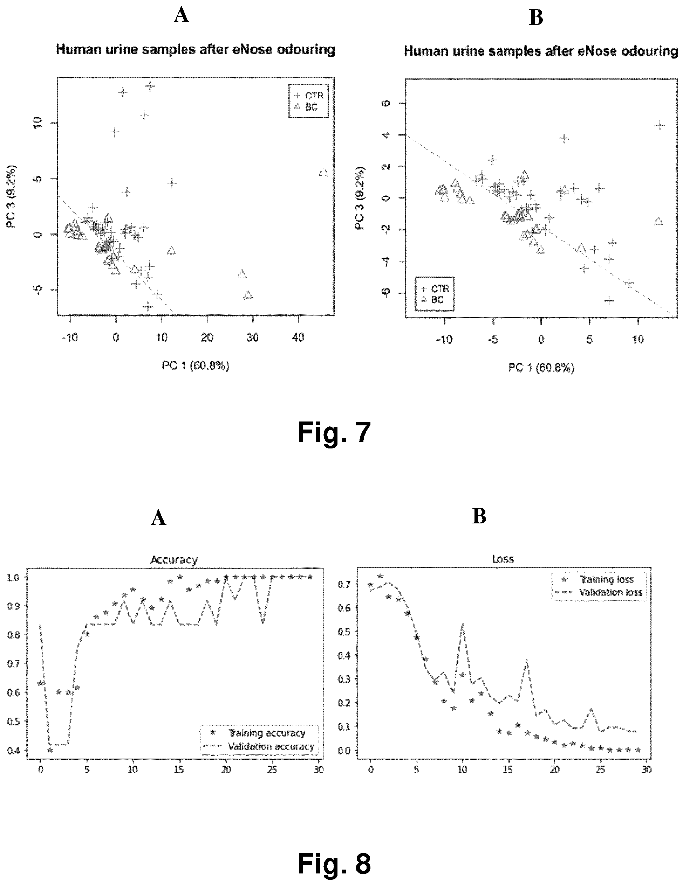

Fig. 8 panels A and B show the accuracy and loss according to the number of samples used to train the CNN model, respectively. Accuracy refers to the proportion of samples that are properly classified from all collected samples. The loss function is the function that computes the distance between the current output of the algorithm and the expected output. Hence, it describes how well the classification algorithm is classifying the samples. Accuracy raises and loss gets lower while more samples are used to train the model. Samples enter the model one batch at a time and the model predicts their class. The loss function is used to calculate how far the prediction is from reality. The model is then adjusted in proportionality to the value of the loss. As a consequence, when the next batch of samples enter the model, its prediction will be more accurate (hence the accuracy will get higher) and the loss value will be lower. These graphs show the evolution of these values when every batch enters the model.
Fig. 9 depicts different interfaces of the software application, according to an embodiment. A) shows a personal information interface (e.g., health data and demographic data of the subject); B) shows a configuration/settings interface; C) and D) show a screening history interface.
Fig. 10 depicts the testing interface which guides the user through different steps to perform the screening. The steps are the following: A) connecting the portable medical screening device to e.g., a computer device; B) collecting a test sample; C) introducing the test sample; D) closing the drawer; E) initiating the screening process; and F) displaying of the screening results and recommendations according to results.

### Detailed Description of Embodiments

The present invention provides a system, a device and a method for screening a disease in a subject, in particular breast cancer.

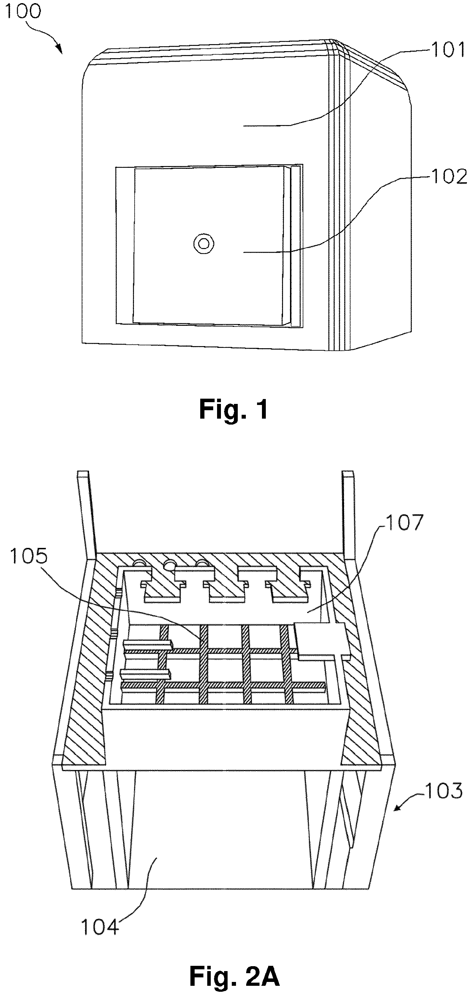

With reference to Fig. 1 and Figs. 2A-C, therein an example of the proposed portable medical screening device (or simply screening device) 100 is shown. According to this embodiment, the screening device 100 is composed of a box body 101, which has a hollow portion for the introduction of a drawer 102. A user/subject can open the drawer 102 and introduce a test sample to be screened/analyzed in a collection chamber 104 of the screening device 100. The user may open and close the drawer 102 by pulling and pushing a knob or other such means attached to the drawer 102. It should be noted that in other embodiments, not shown in the figures, the screening device 100 may not include the drawer 102.

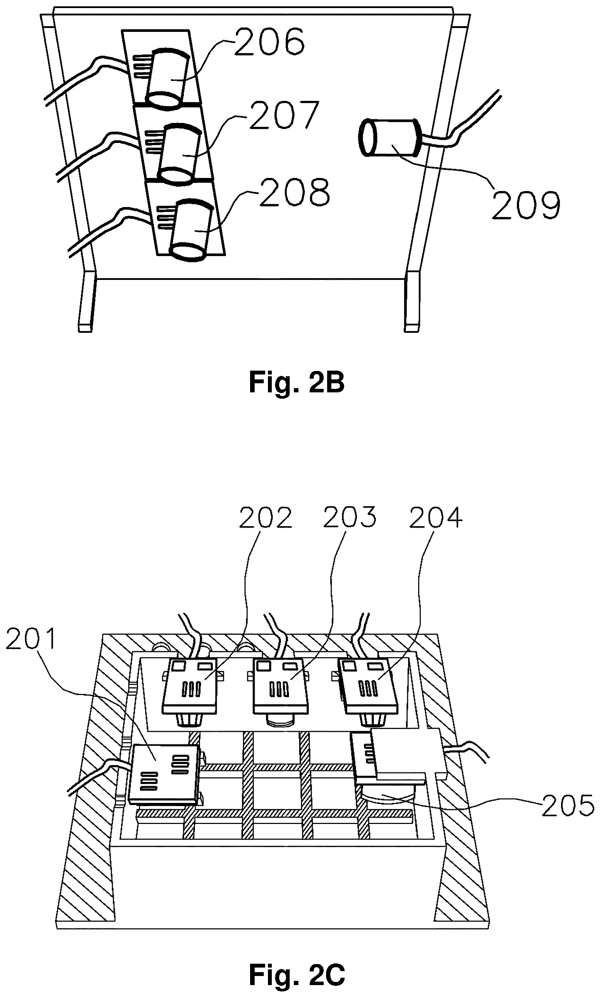

Fig. 2A shows an example of the inner structure 103 of the screening device 100 including a collection chamber 104, a grid 105 (not limitative since in other embodiments the screening device may not have this component), and an analysis chamber 107. The screening device 100 also includes a set of chemically sensitive sensors 201-209, depicted in Fig. 2B and Fig. 2C as a particular embodiment of the present invention, a computational component (or first processing unit) and, optionally, a communication unit. In other embodiments, the set of chemically sensitive sensors can vary in number and type of sensors (particularly, it can include between 5 and 20). The inner structure 103 is contained inside the box body 101.

When a test sample (e.g. a human sample such as urine blood, sweat, vaginal discharge, among others) is introduced into the collection chamber 104, chemical components evaporate from the sample, cross the grid 105 (if present) and arrive to an upper compartment where the analysis chamber 107 is located and where the set of chemically sensitive sensors 201-209, in particular metal oxide sensors (e.g., tin oxide sensors, among others) and/or organic compounds sensitive sensors, can detect VOCs in the test sample and generate an output signal indicative of the presence or absence of such VOCs in the test sample as a result of the detection. Particularly, the generated output signal is an (analog) electric signal whose voltage is proportional to a VOCs concentration.

Particularly, the first processing unit and the communication unit (if present) are placed at a lower part of the screening device 100, within the inner structure 103 and behind the collection chamber 104. The first processing unit is operatively connected, via a wired or a wireless connection, to the set of chemically sensitive sensors 201-209 to receive the generated output signal. The first processing unit in some embodiments can be a microprocessor.

Although not shown, in some embodiments, the drawer 102 (if present) can be filled with an insulating material and comprise a hole to insert the collection chamber containing the test sample to be screened/analyzed therein. Yet, the inner walls of the hole can be additionally coated with a thermal blanket. To control the sample heating process, the first processing unit can be operatively connected to a relay that can mechanically start and stop the device from feeding an electric voltage to the thermal blanket and to a temperature sensor in contact with the hole walls.

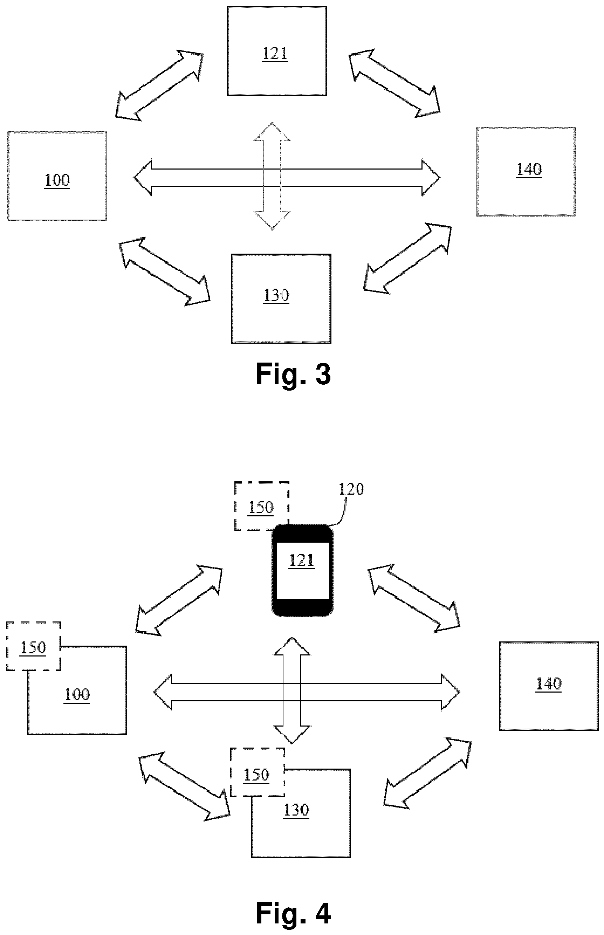

Fig. 3 shows an example of the different elements/entities that can be included in the proposed system for screening a disease, in particular cancer, in a user/subject. The system of this particular embodiment comprises the disclosed screening device 100, a software application 121, which is installed on a user's computer device 120 (see Fig. 4), a remote processing unit 130, and a database (or memory) 140. It should be noted that in other embodiments the system does not need to include all the above-indicated elements. Moreover, in other embodiments, the system can comprise more than one memory/database and the memory/database 140 instead of being remote can be included in any of the other system elements, i.e., the software application 121, the first processing unit, and/or the remote processing unit 130.

As shown in Fig. 3, the screening device 100, the software application 121, the remote processing unit 130, for example a cloud-based server, and the memory or database 140 can be operatively connected and exchange data between them (e.g., the output signal or sensor data, the subject's data, the data stored in the memory or database -health or demographic data of the subject and/or a plurality of healthy and unhealthy individuals or the outcome regarding the disease-). In some embodiments, the screening device 100 is accessed via the user's computer device 120, which is in turn connected to the remote processing unit 130 that can send and receive data from the memory/database 140. Alternatively or complementarily, the data can further flow from the software application 121 to the remote processing unit 130 and from the remote processing unit 130 to the software application 121. Similarly, the data can be assessed directly from the user's computer device 120 connected to the remote processing unit 130 and exchange the information. Further, the remote processing unit 130 can be connected to the memory/database 140 wherein data flow from the remote processing unit 130 to memory/database 140, and vice versa.

Therefore, classification can be done by means of an Al-based classification software 150 on many of the locations of the proposed solution such as at the screening device 100, at the software application 121 and/or at the remote processing unit 130 (see dashed boxes in Fig. 4). The user/subject can see their results on the screening device 100 itself, for instance via an output display, or else at the user's computer device 120 using the software application 121. The user's computer device 120 can be any device such as a smartphone, a laptop computer, a PDA, a tablet computer, a wearable device, or any combination of the aforementioned, among others.

The memory/database 140 can also store health data and/or demographic data of the subject and of a plurality of healthy and/or unhealthy individuals. For example, the health data and/or demographic data of the user/plurality of healthy and/or unhealthy individuals can include age, gender, sex, ethnicity, place of birth/residence, drug consumption (e.g., tobacco habits, alcohol habits, cannabis habits, other toxic habits), eating habits, user's or individual's medical history, family medical history of the subjects, medication intake, family breast cancer history (e.g., mother, sister, daughter, grandmother, aunt), personal cancer history (e.g., breast cancer, lung cancer, ovarian cancer, colorectal cancer, melanoma, bladder cancer, Non-Hodgkin lymphoma, kidney cancer), weight, height, diabetes history, thyroid disorders, polycystic ovarian syndrome, osteoporosis, other endocrine disorder, menarche timing, menstruation, menopause, number of kids delivered to term, number of premature deliveries, number of abortions, number of children of delivered alive, reproductive status (e.g., pregnant, breast feeding, post-partum, on fertility medication), breast disease history (e.g., breast pain during menstrual cycle, hormonal changes, cysts, fibroadenomas, common lumps, nipple discharge, sore, cracked and itchy nipples, inverted nipples), age at first period, age when giving birth for the first time, age when menopause started, number of mammograms and radiographies undertaken, etc.

Particularly, to perform such classification, the present invention provides the cited artificial intelligence-based classification software 150 that consists of a processing algorithm and a classification algorithm that make use of machine learning (ML) and/or Artificial Intelligence (AI) techniques. The classification is based on the nature of the human sample. Therefore, the Al-based classification software 150 can determine an outcome regarding the disease by processing and classifying the generated output signal. In some other embodiments, the Al-based classification software 150 can further determine the outcome regarding the disease considering the health data and/or demographic data of the plurality of healthy and/or unhealthy individuals, and also of the user/subject.

In some embodiments, the outcome regarding the disease consists of an indication about the presence or absence of the disease. For example, the outcome can just be a "0" or a "1" value or a word (e.g., "healthy" or "unhealthy") asserting whether the screening result is good or bad. In other embodiments, the outcome can further indicate a degree of the disease (e.g., level II), a type (or subtype) of the disease (e.g., breast cancer for type of a disease, or e.g., type of breast cancer for subtype of a disease), a recommendation for treatment and even a size of a tumor.

According to a particular embodiment, the Al-based classification software 150 comprises an artificial neural network (ANN) that takes as input the demographic data of the user/subject. This ANN is used as an encoder to extract information about the subject's medical condition and encodes this information in such a way that it is readable and informative to undergo next data processing step. Moreover, a long short-term memory (LSTM) network can be fed with the sensor data (i.e., the generated output signal). An input layer of the LSTM network consists of two types of neurons: 1) Neurons that take as input the encoded subject's data, having been encoded by the ANN; 2) Neurons that take as input the data coming from the chemically sensitive sensors 201-209. The LSTM therefore consists of two separate sets of neurons (two sub-nets). These are fully connected within themselves and with each other as well, i.e., they are essentially a dense network.

In an embodiment, the Al-based classification software 150 is executed by the software application 121. Thus, in this case, data (e.g., the output signal or sensor data) flow from the screening device 100 to the computer device 120. According to this particular embodiment, the outcome generated by the Al-based classification software 150 is displayed in the computer device 120 by means of the software application 121. To generate the outcome, the Al-based classification software 150 can consider the health data and/or demographic data of the plurality of healthy and/or unhealthy individuals, and also of the user/subject, as explained above.

In another embodiment, the Al-based classification software 150 is executed by the remote processing unit 130. Thus, the data (e.g., output signal) flow from the screening device 100 to the remote processing unit 130. Moreover, additional data (e.g., subject's data) collected by the computer device 120 can flow from the latter to the remote processing unit 130. Similarly, data stored in the memory/database 140 can flow from the latter to the remote processing unit 130, and vice versa. The outcome generated by the Al-based classification software 150 can be displayed in the computer device 120 by means of the software application 121, by the remote processing unit 130 itself or by the screening device 100.

The AI-based classification software 150 is executed by the first processing unit, which is located in the screening device 100. In this case, the outcome generated by the Al-based classification software 150 can be directly displayed by the screening device 100, by the computer device 120 or by the remote processing unit 130, depending on the system/method configuration. To generate said outcome, the Al-based classification software 150 can consider the health data and/or demographic data of the plurality of healthy and/or unhealthy individuals, and also of the user/subject, as explained above.

Fig. 5 shows another embodiment of the system. In this case, the first processing unit and the communication unit are implemented in a hardware board 110, which is included in the screening device 100. The two aforementioned components receive the data acquired at the analysis chamber 107 from the sample (i.e., the generated output signal). The data can be classified on the screening device 100 or else sent to the software application 121 or to the remote processing unit 130. Both the software application 121 and the remote processing unit 130 can allocate the Al-based classification software 150. The software application 121 needs to be downloaded before using the screening device 100 and the data obtained from the test sample can be transferred onto the software application 121 as soon as the communication unit receives the data. The software application 121 can subsequently classify the data or send the data to the remote processing unit 130 to be classified therein.

Before making use of the Al-based classification software 150, the system checks for the latest version of the classification software 150 at a version bucket 131. Before any classification, the system makes sure that the latest version of the classification algorithm 150 is installed. The Al-based classification software 150 periodically receives real-time updates. Once a piece of data obtained from a certain test sample is analyzed and classified, these data is stored in the database 140. Further, when the Al-based classification software 150 has determined a diagnosis, this can be displayed, as explained before.

The software application 121 can be developed using Python and can be run on Linux using terminal sessions. The software application 121 may act as a user guide through the screening process. In some embodiments, access to the software application 121 is made via a user login through a given user interface (see Fig. 9 and Fig. 10). The software application 121 may further provide a welcome screen, a menu, a personal data screen and a screening history screen of the application providing various screens a user of the application will see. The software application 121 can be used for acquiring the health data and/or demographic data of the user/subject.

### Examples

### Example 1: Breast cancer screening setup of a first device prototype

The aim was to find a VOCs pattern in both synthetic urine and human urine from control subjects and metastatic breast cancer patients in order to train the screening device prototype to be able to finally screen breast cancer in real human urine samples.

### 1.1 Methods

### 1.1.1 Synthetic urine preparation and human urine collection

6 single-VOC solutions and 30 synthetic urine samples were prepared, and 90 human urine samples were collected.

Firstly, 6 VOC samples were prepared for a first theoretic approach, which contain control VOCs (CTR) (i.e., VOCs usually found in healthy human urine) or breast cancer-related VOCs (BC) in 11 ml distilled water (Table 1).

**Table 1. Single-VOCs samples. 8-oxodG is 8-oxo-2'-deoxyguanosine.**

| **VOC sample** | **Concentration (µg)** |
|---|---|
| Acetone | 8.66 |
| 2-butanone | 0.46 |
| 2-nonanone | 32.62 |
| Benzoic acid | 28 |
| 8-oxodG | 0.430 |
| 8-oxodG | 0.687 |

After single-VOC solution aliquoting, 30 synthetic urine samples were prepared to further test the classification algorithm. With the aim of bringing the synthetic urines as close to reality as possible, 10 urine types were simulated taking different VOC concentration values within expected ranges (Table 2). 11 ml urine solutions contained VOC concentrations corresponding to the expected concentrations one would encounter in 100 ml human urine.

**Table 2. Composition of the synthetic urine. Values are expressed in ml of the samples previously prepared in Table 1.**

| **VOC** | **Control-simulated urine** | | | | **Breast cancer-simulated urine** | | | | | |
|---|---|---|---|---|---|---|---|---|---|---|
| | Urine #1 | Urine #2 | Urine #3 | Urine #4 | Urine #5 | Urine #6 | Urine #7 | Urine #8 | Urine #9 | Urine #10 |
| Acetone | 0.10 | 1.51 | 3.00 | 4.53 | 0.10 | 0.91 | 1.81 | 2.72 | 3.62 | 4.53 |
| 2-butanone | 0.10 | 1.15 | 2.30 | 3.44 | 0.10 | 0.69 | 1.38 | 2.09 | 2.76 | 3.44 |
| 2-nonanone | 0 | 0 | 0 | 0 | 1.09 | 1.26 | 1.44 | 1.61 | 1.78 | 1.96 |
| Benzoic acid | 0.93 | 1.18 | 1.43 | 1.68 | 0 | 0 | 0 | 0 | 0 | 0 |
| 8-oxodG | 1.76 | 1.23 | 0.70 | 0.17 | 4.39 | 3.57 | 2.75 | 1.94 | 1.12 | 0.30 |

Once single-VOC solutions and synthetic urines samples were made, human urine samples were collected. To ensure the anonymity of the participants in the study, each participant was randomly assigned a number to which its samples would be referred during the analysis.

Human sampling was performed to 90 adult women. Patients were excluded if they had a history of any cancer type other than breast cancer, urinary tract infection or other pathology that might affect urine composition. Alcohol consumption was discouraged within 12 h before sample extraction. There were no exclusion criteria regarding ethnicity of the patient and consumption of tobacco, drugs or food. Menstruation was not an exclusion criterion. However, its proximity to the sampling event was recorded to understand any future misclassification that might occur. Aliquots of urine were transferred to 11 ml headspace vials and frozen until analysis. Human urine samples were stored at 0 ºC for 24-48 h.

Breast cancer patients' urine sampling was performed to 39 metastatic breast cancer patients at an advanced stage at Hospital Universitari Sant Joan, Reus (Spain). Their ages ranged 29-75 (mean age 54.9±12.0 y.o.). Patients were randomly selected by the breast cancer oncology team at the hospital. Its protocol, patient consent and legal-ethical aspects were reviewed and approved by the Fundació Institut d'Investigació Sanitària Pere Virgili Ethics Committee (CElm).

Control human urine collection was performed to 51 homogeneously sampled subjects from Tarragona, Barcelona and Reus within the ages 18-78 (mean age 29.7±15.8) that did not present any type of cancer or were not in the knowledge of that. All subjects were excluded from diabetes, cardiovascular diseases and other types of cancer, and gave oral informed consent prior to participation.

### 1.1.2 Gas Chromatography-Mass Spectroscopy (GC-MS) sample analysis

First consideration for Gas Chromatography-Mass Spectroscopy (CG-MS) data inspection was to test whether the VOCs under study were indeed detected by the analysis. GC-MS vacuum setup and machine calibration was performed.

Single-VOC solutions, synthetic urines and human samples were analyzed with a GC-MS for a better understanding of their composition. As for human samples, 51 control samples and 39 breast cancer samples were analyzed 3 times. The GC-MS was programmed to perform 2052 analysis along 20 minutes.

### 1.1.3 Training and testing of the classification algorithm

In this step, previously-obtained sample readings were inspected. The objectives were:
1) To inspect single-VOC solutions' readings to determine if the GC-MS and the device under study were able to properly detect them.
2) To inspect synthetic urines' readings to determine if the GC-MS and the device under study were able to properly discriminate between simulated diseased urines and simulated control urine. In other words, to evaluate whether urine composition was significant when multiple VOC were simultaneously present.
3) To inspect human urine solutions' readings to determine if the GC-MS and the device under study are able to spot a difference between classes.

Once sample preparation and GC-MS analysis were done, samples were classified using pre-processing, processing and prediction algorithms. Input data were raw datafiles from GC-MS that contained odour profile from VOC solutions, synthetic urine and human urine.

Signal pre-processing procedures were implemented with the aim of turning complex raw data into noise-free, readable, significant data. This was attempted by implementing interpolation, chromatogram design, baseline removal, smoothing filtering, normalization and 3-fold sample averaging. This step was done by using R-based code that takes as an input raw GM-MS files and processes them so as to output their total ion chromatograms (TICs).

For the processing step, Python-based processing code was used, which takes as an input the TICs and processes them statistically so as to output sample classification. The algorithm used in this case was Principal component analysis (PCA) and a topological inspection was performed.

Next, the performance of the algorithm was designed for prediction purposes. Their outcomes regarding single-VOC preparations, synthetic samples and human urines were assessed separately. A VOC pattern comparator was obtained to evaluate if GC-MS is able to detect the targeted VOCs, using an R-based code and k-nearest neighbor (kNN) algorithm.

### 1.1.4 Device prototype design and testing

Next step consisted on carrying out the sample procedure as before, but now analyzing urines with the device prototype under study instead of using a GC-MS. A first prototype of the device was designed and built, which is based on an Arduino board with four VOC-chemically sensitive sensors (metal oxide sensors) to capture the "smellprint" of urine, which is referred as TIC. The metal oxide sensors used were the following:
- TGS 2600, Figaro Inc. (sensible to gaseous air contaminants)
- TGS 2602, Figaro Inc. (sensible to VOCs, NH₃, Methylbenzene, among others)
- TGS 2610, Figaro Inc. (sensible to propane, butane, among others)
- TGS 2620, Figaro Inc. (sensible to alcohol, organic vapors, VOCs)

90 human urine samples were odoured by the device prototype and the previously-designed software was implemented into the Arduino microcontroller. Every sample was odoured once by all four chemically sensitive sensors at every second for 30 seconds. Hence, the output of sample recording is a 120-dimensional feature vector for every sample.

Input data to perform the classification was the odour profile of synthetic urines and human samples odoured by the device prototype. The PCA and kNN algorithms were used as templates to design a classification algorithm.

Because the Arduino microprocessor does not have enough storage to host a PCA code, this one was engineered on-line and subsequently implemented into the Arduino, enabling it to perform autonomously off-line through another code implemented into the device. This code contains a classification algorithm that enables the device to classify the sample as control or breast cancer based on the VOCs profile.

Once the algorithm was implemented into the Arduino microprocessor, 54 samples were randomly selected from the whole dataset (n=90) and used to train the algorithm, i.e., to define the scores -parameters- that define the PCA. The remaining 36 test samples were used to test the device prototype by classifying the samples. Finally, in order to validate the classification results obtained by the device prototype, results were compared to those obtained GC-MS data (classification obtained by using PCA and kNN).

### 1.2 Results

### 1.2.1 GC-MS screening and sample classification

This section is dedicated to evaluating the classification success of human urine samples that were analyzed with the GC-MS. As shown in Fig. 6 (A), GC-MS data after dimensionality reduction by PCA visually shows a difference between control (crosses) and metastatic breast cancer (triangles) urine samples. Fig. 6 (B) is a zoomed representation of Fig. 6 (A). Principal components (PCs) 1 and 2 explain a large percentage of the variance among classes. PCs are new features that are constructed as linear combinations or mixtures of the initial features - i.e., of the chemically sensitive sensors' responses at each instant of time. Geometrically speaking, PCs represent the directions of the data that explain a maximal amount of variance, i.e., the lines that capture most information of the data.

The score plot in Fig. 6 shows how 81.7% of samples in quadrant I are controls (top left), 85.7% of samples in quadrants II and III (right) are breast cancer ones and 100% of samples in quadrant IV (bottom left) are also breast cancer. Therefore, it can be stated that dimensionality reduction performs a powerful optimization of sample space variance and that variance explained by the first two PCs might be highly caused by the class to which a given sample belongs.

This procedure aimed to determine whether the GC-MS was sensible enough to discriminate breast cancer samples from control samples - thus obtaining evidence that there existed enough difference between the two classes of samples. Hence, the first step of sample classification was performed with GC-MS data.

After obtaining a score plot built by PCA for human urine samples that were analyzed, a kNN algorithm was also used to obtain another classification. 46 training samples and 44 test samples were used. 11 samples were classified as breast cancer out of 19 actual breast cancer samples. 24 control samples were classified as such out of 25 control samples. Consequently, this precise example performs with a classification rate of 79.55%.

Next point to be considered for model validation is one regarding sample clustering and classification. Because this model manipulates human samples that would influence a patient's treatment and outcome, not only should the classification rate be assessed but also the rate of false negatives (sensitivity) and false positives (specificity). Results are shown in Table 3.

**Table 3. Validation of the classification (PCA) and clustering (kNN) models for human samples regarding classification rate, sensitivity and specificity, when analyzed with the GC-MS.**

| | **PCA (%)** | **kNN (%)** |
|---|---|---|
| Classification rate | 77.11 | 79.55 |
| Sensitivity | 75.05 | 79.04 |
| Specificity | 68.33 | 98.18 |

By relying on the results above, it can be concluded that the kNN classification approach leads to a better class prediction than that of the PCA: not only does kNN excel in classification rate but also in sensitivity, one of the most significant parameters when validating a model with healthcare applications.

### 1.2.2 Device prototype screening

This section is dedicated to evaluating the classification success of human urine samples that were analyzed with the device prototype. Every sample reading was presented in four columns (one per sensor) and 30 rows, since every sensor reads every sample 30 times at every analysis. Once the VOCs patterns were obtained by analyzing the samples through the VOCs chemically sensitive sensors, the resulting data was classified. 54 out of 90 samples were used to train the model and the remaining 36 samples were used to test the model.

A score plot of sample projection against plane PC1-PC3 is shown in greater detail in Fig. 7. By observing this projection, 33 samples gather under the imaginary line, 29 of which (87.9%) are breast cancer samples. Above this threshold there lay 40 control samples, 85.1% of which are control ones. Breast cancer samples were found to be significantly different from control samples.

In Table 4 the validation results of the training model are shown.

**Table 4. Validation of the classification (PCA) training model for human samples (N=90) regarding classification rate, sensitivity and specificity when analyzed with the device.**

| | **PCA (%)** |
|---|---|
| Classification rate | 58.3 |
| Sensitivity | 75.0 |
| Specificity | 45.0 |

From the testing samples, the number of true positives (TP), true negatives (TN), false positives (FP) and false negatives (FN) were 12, 9, 11 and 4, respectively.

This early model of odour-based cancer screening device was further developed in Example 2.

### Example 2: Breast cancer screening by using an improved device prototype

### 2.1 Methods

### 2.1.1 Data collection and processing

The working example was carried out using the extracted data from Example 1 (raw data obtained from the four chemically sensitive sensors - not yet pre-processed). Therefore, data of 51 urine samples from control subjects and 39 urine samples from breast cancer subjects were analyzed and classified using an improved classification algorithm.

Before classifying the data, data was pre-processed so as to ensure that the classification will later on rely on the characteristic of cancer instead of on irrelevant artifacts. Firstly, data was normalized.

### 2.1.2 Software design and testing

After sample pre-processing, data was fed to a convolutional neural network (cNN). This net consisted of four convolutional filters of size 32, 64, 128, 128 and linear activation. The output layer of the neural network presented two cells (two outputs) with a softmax activation function. Specifically, this model was trained with 65 urine samples, tested with 12 samples and validated with 13 samples.

Other classification methods were also implemented, such as PCA, decision tree, kNN and kNN with PCA.

### 2.1.3 Improved device prototype design and testing

As compared to the previous prototype, this second device had 9 VOC chemically sensitive sensors and a more powerful microprocessor. The statistics-based algorithm was also replaced by an Al algorithm. The previous microprocessor was an Arduino UNO whereas this second prototype had a MKR Arduino WiFi, which included WiFi connectivity. Additionally, the classification algorithm of this prototype run in the cloud-based server, which allows for more computational capacity that the previous prototype, in which the classification algorithm is running on edge (on the Arduino board).

### 2.2 Results

As shown in Fig. 8 (A), the accuracy of the cNN model (y-axis) increases as it gets trained as more samples are used to train it -each sample being used multiple times (x-axis). Because accuracy follows a similar fashion in both the training ("*" line) and the testing ("-" line) dataset, it can be concluded that the model is not overfitted. A similar logic (but this time applied to loss function) can be applied to the loss function plot (Fig. 8 (B)): the loss function decreases -hence the classification gets better- as the model is trained. Numerical results are displayed in Table 5.

**Table 5. Training, testing and validation of the cNN classification model for human samples regarding accuracy and loss.**

| | **Training (N=65)** | **Testing (N=12)** | **Validation (N=13)** |
|---|---|---|---|
| Accuracy | 1.0 | 1.0 | 1.0 |
| Loss | 0.0019 | 0.0742 | 0.0230 |

Other classification methods (PCA, decision tree, kNN & kNN with PCA) were also implemented, but none of these methods performed better than the Al model (Table 6).

**Table 6. Comparison of the detectability capacity of various biostatistics methods (PCA, decision tree, kNN and combinations of the latter) versus an Al-based method (convolutional neural networks).**

| | **PCA** | **Decision tree** | **Decision tree with PCA** | **kNN** | **kNN with PCA** | **cNN** |
|---|---|---|---|---|---|---|
| Classification rate (%) | 77.11 | 78.26 | 69.56 | 78.26 | 82.60 | 100.00 |

Throughout the description and claims the word "comprise" and "include" and its variations are not intended to exclude other technical features, additives, components, or steps. Additional objects, advantages and features of the invention will become apparent to those skilled in the art upon examination of the description or may be learned by practice of the invention. Furthermore, the present invention covers all possible combinations of particular and preferred embodiments described herein.

Although the present embodiments have been described with reference to specific example embodiments, it will be evident that various modifications and changes may be made to these embodiments without departing from the broader scope of the appended claims.

## Claims

1. A system for screening a disease in a sample of a human subject, the system comprising:
- a portable medical screening device (100) comprising:
a collection chamber (104) configured to collect at least one test sample of the human subject, wherein the test sample comprises urine;
a set of chemically sensitive sensors (201-209) comprising volatile organic compound-sensitive sensors, including tin oxide sensors, wherein the sensors detect volatile organic compounds in the test sample and generate an output signal indicative of the presence or absence of volatile organic compounds in the test sample as a result of the detection;
an analysis chamber (107) configured to allocate the set of chemically sensitive sensors (201-209);
a first processing unit operatively connected to the set of chemically sensitive sensors (201-209) to receive the generated output signal; and
a communication unit operatively connected to the first processing unit and configured to establish a communication with a remote processing unit (130) and with a software application (121) installed on a computer device (120);
- a software application (121) installed on a computer device (120) which collects health data and/or demographic data of the subject and displays an outcome regarding the disease via a user interface;
- a remote processing unit (130) which executes an artificial intelligence-based classification software (150) configured to determine the outcome regarding the disease,
wherein the artificial intelligence-based classification software (150) is selected from the group consisting of long short-term memory, convolutional neural network, linear discriminant analysis and K-nearest neighbor; and
- a memory or database (140) comprising health data and/or demographic data of the subject and/or of a plurality of healthy and/or unhealthy individuals;
wherein the artificial intelligence-based classification software (150) merges the generated output signal and the health data and/or demographic data of the subject and recognizes patterns among the information stored in the memory or database (140), to generate the outcome regarding the disease, and
wherein the outcome regarding the disease comprises an indication regarding the presence or absence of the disease, wherein the disease is breast cancer.

2. The system according to claim 1, wherein the portable medical screening device (100) is composed of a box body (101) adapted to enclose the collection chamber (104), a grid (105) positioned above the collection chamber (104), the analysis chamber (107), the first processing unit and the communication unit, the box body (101) having a hollow portion for the introduction of a drawer (102).

3. The system of any one of the previous claims, wherein the health data and/or demographic data of the subject is further stored in the memory or database (140), and wherein the artificial intelligence-based classification software (150) considers the stored health data and/or demographic data of the subject to determine the outcome regarding the disease.

4. The system of any one of the previous claims, wherein the determined outcome regarding the disease is further stored in the memory or database (140), and wherein the artificial intelligence-based classification software (150) determines the outcome regarding the disease considering the stored outcome.

5. The system of any one of the previous claims, wherein health data and/or demographic data of the subject and of the plurality of healthy and unhealthy individuals is selected from the group consisting of age, sex, gender, ethnicity, place of birth/residence, weight, height, body mass index, medication intake, drug consumption, medical history, family medical history, cancer history, family cancer history, breast disease history, family breast cancer history, number of mammograms and radiographies undertaken, diabetes history, thyroid disorders, polycystic ovarian syndrome, osteoporosis, other endocrine disorder, menstruation, menarche and menopause characteristics, reproductive status, number of kids delivered to term, number of premature labors, number of abortions, number of living children, number of children of delivered alive, age when giving birth for the first time, diet type, eating habits, and combinations thereof.

6. The system of any one of the previous claims, wherein the remote processing unit (130) is a cloud-based server.

7. The system of any one of the previous claims, wherein the the software application (121) is located in a smartphone or a web-browser.

8. A method for screening a disease in a sample of a human subject, the method comprising:
collecting, by a collection chamber (104) of a portable medical screening device (100), at least one test sample of the human subject, wherein the test sample comprises urine;
detecting, by a set of chemically sensitive sensors (201-209) comprising volatile organic compound-sensitive sensors, including tin oxide sensors, which are allocated on an analysis chamber (107) of the portable medical screening device (100), volatile organic compounds in the test sample and generating an output signal indicative of the presence or absence of volatile organic compounds in the test sample as a result of the detection;
receiving, by a first processing unit of the portable medical screening device (100), the generated output signal;
establishing, by a communication unit operatively connected to the first processing unit, a communication with a remote processing unit (130) and with a software application (121) installed on a computer device (120);
collecting, by a software application (121) installed on a computer device (120), health data and/or demographic data of the subject via a user interface;
determining, by an artificial intelligence-based classification software (150) executed by a remote processing unit (130), an outcome regarding the disease by merging the generated output signal and the health data and/or demographic data of the subject and recognizes patterns among the information stored in a memory or database (140) comprising health data and/or demographic data of a plurality of healthy and unhealthy individuals,
wherein the artificial intelligence-based classification software (150) is selected from the group consisting of long short-term memory, convolutional neural network, linear discriminant analysis and K-nearest neighbor; and
displaying, by the user interface of the software application (121) installed on a computer device (120), the outcome regarding the disease,
wherein the outcome regarding the disease comprises an indication regarding the presence or absence of the disease, wherein the disease is breast cancer.

## Patentansprüche

1. System zum Screening einer Krankheit in einer Probe eines menschlichen Probanden, wobei das System Folgendes umfasst:
- eine tragbare medizinische Screening-Vorrichtung (100), umfassend:
eine Sammlungskammer (104), die dazu konfiguriert ist, mindestens eine Testprobe des menschlichen Probanden zu sammeln, wobei die Testprobe Urin umfasst;
einen Satz chemisch empfindlicher Sensoren (201-209), umfassend für flüchtige organische Verbindungen empfindliche Sensoren, einschließlich Zinnoxidsensoren, wobei die Sensoren flüchtige organische Verbindungen in der Testprobe erkennen und als Ergebnis der Erkennung ein Ausgangssignal generieren, das die Anwesenheit oder Abwesenheit flüchtiger organischer Verbindungen in der Testprobe angibt;
eine Analysekammer (107), die dazu konfiguriert ist, den Satz chemisch empfindlicher Sensoren (201-209) aufzunehmen;
eine erste Verarbeitungseinheit, die betriebsmäßig mit dem Satz chemisch empfindlicher Sensoren (201-209) verbunden ist, um das generierte Ausgangssignal zu empfangen; und
eine Kommunikationseinheit, die betriebsmäßig mit der ersten Verarbeitungseinheit verbunden und dazu konfiguriert ist, eine Kommunikation mit einer entfernten Verarbeitungseinheit (130) und mit einer auf einer Computervorrichtung (120) installierten Softwareanwendung (121) herzustellen;
- eine auf einer Computervorrichtung (120) installierte Softwareanwendung (121), die Gesundheitsdaten und/oder demografische Daten des Probanden sammelt und über eine Benutzeroberfläche ein Ergebnis bezüglich der Krankheit anzeigt;
- eine entfernte Verarbeitungseinheit (130), die eine auf künstlicher Intelligenz basierende Klassifizierungssoftware (150) ausführt, die dazu konfiguriert ist, das Ergebnis bezüglich der Krankheit zu bestimmen,
wobei die auf künstlicher Intelligenz basierende Klassifizierungssoftware (150) aus der Gruppe bestehend aus langem Kurzzeitgedächtnis, neuronalem Faltungsnetzwerk, linearer Diskriminanzanalyse und K-nächsten-Nachbarn ausgewählt ist; und
- einen Speicher oder eine Datenbank (140), umfassend Gesundheitsdaten und/oder demografische Daten des Probanden und/oder einer Vielzahl von gesunden und/oder ungesunden Individuen;
wobei die auf künstlicher Intelligenz basierende Klassifizierungssoftware (150) das generierte Ausgangssignal und die Gesundheitsdaten und/oder demografischen Daten des Probanden zusammenführt und Muster unter den im Speicher oder in der Datenbank (140) gespeicherten Informationen erkennt, um das Ergebnis bezüglich der Krankheit zu generieren, und
wobei das Ergebnis bezüglich der Krankheit eine Angabe bezüglich der Anwesenheit oder Abwesenheit der Krankheit umfasst, wobei die Krankheit Brustkrebs ist.

2. System nach Anspruch 1, wobei die tragbare medizinische Screening-Vorrichtung (100) aus einem Gehäusekörper (101), der dazu angepasst ist, die Sammlungskammer (104) zu umschließen, einem Gitter (105), das über der Sammlungskammer (104) positioniert ist, der Analysekammer (107), der ersten Verarbeitungseinheit und der Kommunikationseinheit besteht, wobei der Gehäusekörper (101) einen hohlen Abschnitt zum Einsetzen einer Schublade (102) aufweist.

3. System nach einem der vorhergehenden Ansprüche, wobei die Gesundheitsdaten und/oder demografischen Daten des Probanden zusätzlich in dem Speicher oder der Datenbank (140) gespeichert werden und wobei die auf künstlicher Intelligenz basierende Klassifizierungssoftware (150) die gespeicherten Gesundheitsdaten und/oder demografischen Daten des Probanden berücksichtigt, um das Ergebnis bezüglich der Krankheit zu bestimmen.

4. System nach einem der vorhergehenden Ansprüche, wobei das bestimmte Ergebnis bezüglich der Krankheit ferner in dem Speicher oder der Datenbank (140) gespeichert wird und wobei die auf künstlicher Intelligenz basierende Klassifizierungssoftware (150) das Ergebnis bezüglich der Krankheit unter Berücksichtigung des gespeicherten Ergebnisses bestimmt.

5. System nach einem der vorhergehenden Ansprüche, wobei Gesundheitsdaten und/oder demografische Daten des Probanden und einer Vielzahl von gesunden und ungesunden Individuen aus der Gruppe bestehend aus Alter, Geschlecht, Geschlechtsidentität, ethnischer Zugehörigkeit, Geburtsort/Wohnort, Gewicht, Körpergröße, Körpermasseindex, Medikamenteneinnahme, Drogenkonsum, medizinischer Vorgeschichte, familiärer medizinischer Vorgeschichte, Krebsvorgeschichte, familiärer Krebsvorgeschichte, Brustkrankheitsvorgeschichte, familiärer Brustkrebsvorgeschichte, Anzahl der durchgeführten Mammographien und Röntgenaufnahmen, Diabetesvorgeschichte, Schilddrüsenerkrankungen, polyzystisches Ovarialsyndrom, Osteoporose, andere endokrine Erkrankungen, Merkmale der Menstruation, Menarche und Menopause, Reproduktionsstatus, Anzahl der voll ausgetragenen Kinder, Anzahl der Frühgeburten, Anzahl der Schwangerschaftsabbrüche, Anzahl der lebenden Kinder, Anzahl der lebend geborenen Kinder, Alter bei der ersten Geburt, Ernährungsweise, Essgewohnheiten und Kombinationen davon ausgewählt sind.

6. System nach einem der vorhergehenden Ansprüche, wobei die entfernte Verarbeitungseinheit (130) ein cloudbasierter Server ist.

7. System nach einem der vorhergehenden Ansprüche, wobei sich die Softwareanwendung (121) auf einem Smartphone oder in einem Webbrowser befindet.

8. Verfahren zum Screening einer Krankheit in einer Probe eines menschlichen Probanden, wobei das Verfahren Folgendes umfasst:
Sammeln, durch eine Sammlungskammer (104) einer tragbaren medizinischen Screening-Vorrichtung (100), mindestens einer Probe des menschlichen Probanden, wobei die Testprobe Urin umfasst;
Erkennen, durch einen Satz chemisch empfindlicher Sensoren (201-209), umfassend für flüchtige organische Verbindungen empfindliche Sensoren, einschließlich Zinnoxidsensoren, die an einer Analysekammer (107) der tragbaren medizinischen Screening-Vorrichtung (100) angeordnet sind, von flüchtigen organischen Verbindungen in der Testprobe und Generieren, als Ergebnis der Erkennung, eines Ausgangssignals, das die Anwesenheit oder Abwesenheit flüchtiger organischer Verbindungen in der Testprobe angibt;
Empfangen des generierten Ausgangssignals durch eine erste Verarbeitungseinheit der tragbaren medizinischen Screening-Vorrichtung (100);
Herstellen, durch eine Kommunikationseinheit, die betriebsmäßig mit der ersten Verarbeitungseinheit verbunden ist, einer Kommunikation mit einer entfernten Verarbeitungseinheit (130) und mit einer auf einer Computervorrichtung (120) installierten Softwareanwendung (121);
Sammeln, durch eine auf einer Computervorrichtung (120) installierte Softwareanwendung (121), von Gesundheitsdaten und/oder demografischen Daten des Probanden über eine Benutzeroberfläche;
Bestimmen, durch eine auf künstlicher Intelligenz basierende Klassifizierungssoftware (150), die von einer entfernten Verarbeitungseinheit (130) ausgeführt wird, eines Ergebnisses bezüglich der Krankheit durch Zusammenführen des generierten Ausgangssignals und der Gesundheitsdaten und/oder demografischen Daten des Probanden und Erkennen von Mustern unter den in einem Speicher oder einer Datenbank (140) gespeicherten Informationen, umfassend Gesundheitsdaten und/oder demografische Daten einer Vielzahl von gesunden und ungesunden Individuen,
wobei die auf künstlicher Intelligenz basierende Klassifizierungssoftware (150) aus der Gruppe bestehend aus langem Kurzzeitgedächtnis, neuronalem Faltungsnetzwerk, linearer Diskriminanzanalyse und K-nächsten-Nachbarn ausgewählt ist; und
Anzeigen des Ergebnisses bezüglich der Krankheit über die Benutzeroberfläche der auf einer Computervorrichtung (120) installierten Softwareanwendung (121),
wobei das Ergebnis bezüglich der Krankheit eine Angabe bezüglich der Anwesenheit oder Abwesenheit der Krankheit umfasst, wobei die Krankheit Brustkrebs ist.

## Revendications

1. Système de dépistage d'une maladie dans un échantillon d'un sujet humain, le système comprenant :
un dispositif portable de dépistage médical (100) comprenant :
une chambre de collecte (104) configurée pour collecter au moins un échantillon d'essai du sujet humain, dans lequel l'échantillon d'essai comprend de l'urine ;
un ensemble de capteurs chimiquement sensibles (201-209) comprenant des capteurs sensibles aux composés organiques volatils, y compris des capteurs à oxyde d'étain, dans lequel les capteurs détectent des composés organiques volatils dans l'échantillon d'essai et génèrent un signal de sortie indicatif de la présence ou de l'absence de composés organiques volatils dans l'échantillon d'essai à la suite de la détection ;
une chambre d'analyse (107) configurée pour loger l'ensemble de capteurs chimiquement sensibles (201-209) ;
une première unité de traitement reliée de manière opérationnelle à l'ensemble de capteurs chimiquement sensibles (201-209) pour recevoir le signal de sortie généré ; et
une unité de communication reliée de manière opérationnelle à la première unité de traitement et configurée pour établir une communication avec une unité de traitement distante (130) et avec une application logicielle (121) installée sur un dispositif informatique (120) ;
- une application logicielle (121) installée sur un dispositif informatique (120) qui collecte des données de santé et/ou des données démographiques du sujet et affiche un résultat concernant la maladie via une interface utilisateur ;
- une unité de traitement distante (130) qui exécute un logiciel de classification basé sur l'intelligence artificielle (150) configuré pour déterminer le résultat concernant la maladie,
- dans lequel le logiciel de classification basé sur l'intelligence artificielle (150) est sélectionné dans le groupe constitué de mémoire à long et court terme, réseau neuronal convolutionnel, analyse discriminante linéaire et k plus proches voisins ; et
- une mémoire ou base de données (140) comprenant des données de santé et/ou des données démographiques du sujet et/ou d'une pluralité d'individus sains et/ou malades ;
dans lequel le logiciel de classification basé sur l'intelligence artificielle (150) fusionne le signal de sortie généré et les données de santé et/ou les données démographiques du sujet et reconnaît des motifs parmi les informations stockées dans la mémoire ou base de données (140), afin de générer le résultat concernant la maladie, et
dans lequel le résultat concernant la maladie comprend une indication relative à la présence ou à l'absence de la maladie, dans lequel la maladie est un cancer du sein.

2. Le système selon la revendication 1, dans lequel le dispositif portable de dépistage médical (100) est constitué d'un corps de boîtier (101) agencé pour renfermer la chambre de collecte (104), une grille (105) positionnée au-dessus de la chambre de collecte (104), la chambre d'analyse (107), la première unité de traitement et l'unité de communication, le corps de boîtier (101) comportant une partie creuse pour l'introduction d'un tiroir (102).

3. Le système selon l'une quelconque des revendications précédentes, dans lequel les données de santé et/ou les données démographiques du sujet sont en outre stockées dans la mémoire ou base de données (140), et dans lequel le logiciel de classification basé sur l'intelligence artificielle (150) prend en compte les données de santé et/ou les données démographiques du sujet stockées pour déterminer le résultat concernant la maladie.

4. Le système selon l'une quelconque des revendications précédentes, dans lequel le résultat déterminé concernant la maladie est en outre stocké dans la mémoire ou base de données (140), et dans lequel le logiciel de classification basé sur l'intelligence artificielle (150) détermine le résultat concernant la maladie en tenant compte du résultat stocké.

5. Le système selon l'une quelconque des revendications précédentes, dans lequel les données de santé et/ou les données démographiques du sujet et de la pluralité d'individus sains et/ou malades sont sélectionnées dans le groupe constitué de l'âge, le sexe, le genre, l'origine ethnique, le lieu de naissance/de résidence, le poids, la taille, l'indice de masse corporelle, la prise de médicaments, la consommation de drogues, les antécédents médicaux, les antécédents médicaux familiaux, les antécédents de cancer, les antécédents familiaux de cancer, les antécédents de maladie mammaire, les antécédents familiaux de cancer du sein, le nombre de mammographies et de radiographies effectuées, les antécédents de diabète, les troubles thyroïdiens, le syndrome des ovaires polykystiques, l'ostéoporose, d'autres troubles endocriniens, la menstruation, les caractéristiques de la ménarche et de la ménopause, le statut reproductif, le nombre d'enfants nés à terme, le nombre d'accouchements prématurés, le nombre d'avortements, le nombre d'enfants vivants, le nombre d'enfants nés vivants, l'âge lors du premier accouchement, le type de régime alimentaire, les habitudes alimentaires, et leurs combinaisons.

6. Le système selon l'une quelconque des revendications précédentes, dans lequel l'unité de traitement distante (130) est un serveur basé sur le cloud.

7. Le système selon l'une quelconque des revendications précédentes, dans lequel l'application logicielle (121) est située dans un smartphone ou un navigateur web.

8. Procédé de dépistage d'une maladie dans un échantillon d'un sujet humain, le procédé comprenant :
collecter, au moyen d'une chambre de collecte (104) d'un dispositif portable de dépistage médical (100), au moins un échantillon d'essai du sujet humain, dans lequel l'échantillon d'essai comprend de l'urine ;
détecter, au moyen d'un ensemble de capteurs chimiquement sensibles (201-209) comprenant des capteurs sensibles aux composés organiques volatils, y compris des capteurs à oxyde d'étain, qui sont agencés dans une chambre d'analyse (107) du dispositif portable de dépistage médical (100), des composés organiques volatils dans l'échantillon d'essai et générer un signal de sortie indicatif de la présence ou de l'absence de composés organiques volatils dans l'échantillon d'essai à la suite de la détection ;
recevoir, au moyen d'une première unité de traitement du dispositif portable de dépistage médical (100), le signal de sortie généré ;
établir, au moyen d'une unité de communication reliée de manière opérationnelle à la première unité de traitement, une communication avec une unité de traitement distante (130) et avec une application logicielle (121) installée sur un dispositif informatique (120) ;
collecter, au moyen d'une application logicielle (121) installée sur un dispositif informatique (120), des données de santé et/ou des données démographiques du sujet via une interface utilisateur ;
déterminer, au moyen d'un logiciel de classification basé sur l'intelligence artificielle (150) exécuté par une unité de traitement distante (130), un résultat concernant la maladie en fusionnant le signal de sortie généré et les données de santé et/ou les données démographiques du sujet et en reconnaissant des motifs parmi les informations stockées dans une mémoire ou base de données (140) comprenant des données de santé et/ou des données démographiques d'une pluralité d'individus sains et malades,
dans lequel le logiciel de classification basé sur l'intelligence artificielle (150) est sélectionné dans le groupe constitué de mémoire à long et court terme, réseau neuronal convolutionnel, analyse discriminante linéaire et k plus proches voisins ; et
afficher, au moyen de l'interface utilisateur de l'application logicielle (121) installée sur un dispositif informatique (120), le résultat concernant la maladie,
dans lequel le résultat concernant la maladie comprend une indication relative à la présence ou à l'absence de la maladie, dans lequel la maladie est un cancer du sein.
